# EUROPEAN PATENT APPLICATION

(11) **EP 0 820 994 A1**
(43) Date of publication of application: **28.01.1998**
(21) Application number: 97111452.5
(22) Date of filing: 07.07.1997
(51) Int. Cl.: C07D 257/04, C07D 401/06, C07D 403/06, A01N 47/38

(54) **Tetrazolinones as herbicides and intermediates**

(30) Priority: 18.07.1996 JP 206416/96
(71) Applicant: NIHON BAYER AGROCHEM K.K., Tokyo 108 (JP)
(72) Inventor: Goto, Toshio, Shimotsuga-gun, Tochigi (JP); Ito, Seishi, Oyama-shi, Tochigi (JP); Minegishi, Natsuko, Oyama-shi, Tochigi (JP); Yamaoka, Tatsuya, Oyama-shi, Tochigi (JP); Kitagawa, Yoshinori, Mouka-shi, Tochigi (JP); Shibuya, Katsuhiko, Kawachi-gun, Tochigi (JP)
(74) Representative: Linkenheil, Dieter, Dr.

(57) **Abstract**

The present invention relates to novel compounds and mixtures of geometrical isomers, which are represented by the formula: 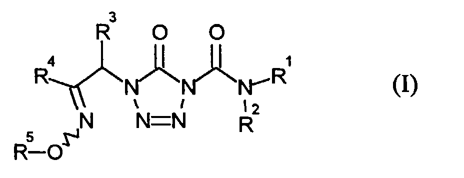 wherein
- R¹ and R²: independently of one another are C₁₋₆ alkyl, C₁₋₆ haloalkyl, C₃₋₈ cycloalkyl, C₂₋₆ alkenyl, C₂₋₆ haloalkenyl, C₃₋₆ alkynyl or optionally substituted phenyl; or R¹ and R², together with the nitrogen atom to which they are bonded, form a 5-membered or 6-membered heterocyclic ring which may be benzo-fused or which may be substituted by halogen or C₁₋₄ alkyl; R³ is hydrogen or C₁₋₆ alkyl; R⁴ is hydrogen or C₁₋₆ alkyl; or R³ and R⁴, together with the carbon atoms to which they are bonded, form cyclopentylidene or cyclohexylidene; and R⁵ is C₁₋₆ alkyl, C₃₋₆ alkenyl or benzyl;
further to processes and new intermediates for their production and to their use as herbicides.

## Description

The present invention relates to novel tetrazolinones, processes for their preparation and their use as herbicides, as well as new intermediates for their preparation.

It has been already known that certain tetrazolinone derivatives have herbicidal activities [see EP-OS (European Patent Laid-Open Specification) 146 279 (= US-Patents 4,618,365, 4,826,529, 4,830,661, 4,956,469, 5,003,075 and 5,019,152), Japanese Patent Kokai Publications Hei 5-331153, Hei 5-331154, Hei 5-339249, Hei 6-199818 and Hei 6-30601].

There have been found novel compounds and mixtures of geometrical isomers, of the formula (I): 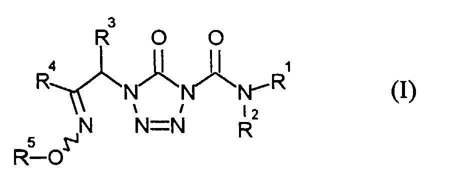 wherein
R¹ and R², independently of one another, are C₁₋₆ alkyl, C₁₋₆ haloalkyl, C₃₋₈ cycloalkyl, C₂₋₆ alkenyl, C₂₋₆ haloalkenyl, C₃₋₆ alkynyl or optionally substituted phenyl, or
R¹ and R², together with the nitrogen atom to which they are bonded, form a 5-membered or 6-membered heterocyclic ring which may be benzo-fused or which may be substituted by halogen or C₁₋₄ alkyl,
R³ is hydrogen or C₁₋₆ alkyl,
R⁴ is hydrogen or C₁₋₆ alkyl, or
R³ and R⁴, together with the carbon atoms to which they are bonded, form cyclopentylidene or cyclohexylidene,
R⁵ is C₁₋₆ alkyl, C₃₋₆ alkenyl or benzyl, and
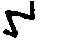 is a single bond of Anti form or of Syn form.

The compounds of the above formula (I) according to the invention can be obtained, for example, by processes in which
(a):
   compounds of the formula (II): 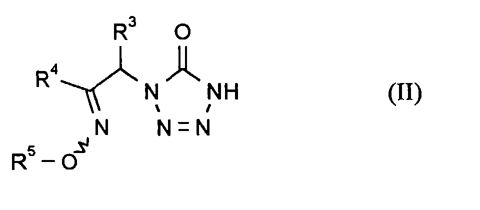 wherein
   - R³, R⁴ and R⁵: have the above-mentioned meanings,
   are reacted with compounds of the formula (III): 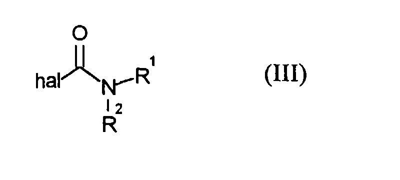 wherein
   - R¹ and R²: have the above-mentioned meanings, and
   - hal: represents a leaving group such as a chlorine atom or bromine atom,
   in the presence of inert solvents, and if appropriate, in the presence of an acid binder,
   or
(b)
   compounds of the formula (IV): 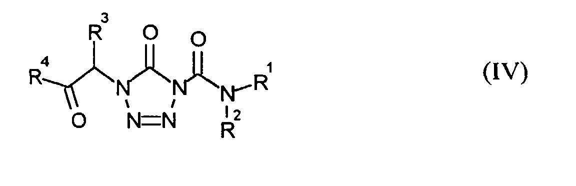 wherein
   - R¹, R², R³ and R⁴: have the above-mentioned meanings,
   are reacted with compounds of the formula (V):

   R⁵―O―NH₂ (V)

   wherein R⁵ has the above-mentioned meaning,
   in the presence of inert solvents, and if appropriate, in the presence of an acid binder.

The compounds of the formula (I) according to the invention exhibit strong herbicidal activity.

Surprisingly, the compounds of the formula (I) according to the invention exhibit excellent, superior herbicidal action in comparison with the previously known compounds concretely described in the above prior art publications EP-A 146 279 (= USPs 4,618,365, 4,826,529, 4,830,661, 4,956,469, 5,003,075 and 5,019,152), JP-A Hei 5-331153, Hei 5-331154, Hei 5-339249, Hei 6-199818 and Hei 6-30601.

In each of the formulae concerning the compounds of the formula (I) and intermediates for their preparation according to the invention, the term "halogen" in halogen, haloalkyl and haloalkoxy represents fluoro, chloro, bromo and iodo, preferably being chloro or fluoro.

The "alkyl" may be straight-chain or branched, and includes, for example, methyl, ethyl, propyl, isopropyl, n-, iso-, sec- or tert-butyl, n-, iso-, sec-, tert- or neo-pentyl, and n-, iso-, sec-, tert- or neo-hexyl.

The "haloalkyl" represents the above alkyl which is substituted by halogen(s), and when the haloalkyl is substituted by plural halogens, these halogens may be same or different. Examples thereof include, for example, trifluoromethyl, 2-chloroethyl and 2,2,2-trifluoroethyl.

The "cycloalkyl" includes, for example, cyclopropyl, cyclobutyl, cyclopentyl, cyclohexyl, cycloheptyl and cyclooctyl.

The "alkenyl" may be straight-chain or branched, and includes, for example, vinyl, allyl, isopropenyl, 1-methyl-2-propenyl, 2-(3-)butenyl and 2-(3-, 4-)pentenyl. The "haloalkenyl" represents the above alkenyl which is substituted by halogen(s), and when the haloalkenyl is substituted by plural halogens, these halogens may be identical or different. Examples thereof include, for example, 2-chloro-2-propenyl.

The "alkynyl" includes, for example, propargyl.

The "phenyl" may optionally be substituted. Examples of the substituents thereof include, for example, halogen such as chloro, fluoro and bromo; cyano; nitro; alkyl such as methyl, ethyl, propyl and isopropyl; haloalkyl such as trifluoromethyl; alkoxy such as methoxy and ethoxy; haloalkoxy such as trifluoromethoxy; and alkylthio such as methylthio.

The "5-membered or 6-membered heterocyclic ring" represents a 5-membered or 6-membered heterocyclic ring which contains at least one nitrogen atom as a hetero atom and which may contain further hetero atom(s) arbitrarily selected from a nitrogen atom, an oxygen atom or a sulfur atom. Said heterocyclic ring may optionally be substituted by halogen such as fluoro, chloro and bromo, or by C₁₋₄ alkyl such as methyl, ethyl, propyl and isopropyl, or may be benzo-fused. Examples of the 5-membered or 6-membered heterocyclic rings include pyrrolidinyl, 2,5-dimethylpyrrolidinyl, pyrrolinyl, 2,5-dimethylpyrrolinyl, imidazolidinyl, pyrazolidinyl, pyrazolinyl, piperidyl, 2-methylpiperidyl, 2,6-dimethylpiperidyl, piperazinyl, indolinyl, morpholinyl, 1,2,3,4-tetrahydroquinolyl, 2-methyl-1,2,3,4-tetrahydroquinolyl, 2,2-dimethyl-1,2,3,4-tetrahydroquinolyl, 2,2-dimethyl-1,2-dihydroquinolyl and 6-fluoro-2,2-dimethyl-1,2-dihydroquinolyl.

The "alkoxy" may be straight-chain or branched, and includes, for example, methoxy, ethoxy, propoxy, isopropoxy, n-, iso-, sec- or tert-butoxy, n-, iso-, sec-, tert- or neo-pentoxy, and n-, iso-, sec-, tert- or neo-hexoxy.

Among the compounds and mixtures of geometrical isomers of the formula (I) according to the invention, preferred compounds are those
wherein,
R¹ and R², independently of one another, are C₁₋₄ alkyl, C₁₋₄ haloalkyl, cyclopropyl, cyclopentyl, cyclohexyl, C₂₋₄ alkenyl, C₂₋₄ haloalkenyl or C₃₋₄ alkynyl, or represent phenyl which may be substituted by halogen or C₁₋₄ alkyl, or
R¹ and R², together with the nitrogen atom to which they are bonded, form pyrrolidin-1-yl, 2,5-dimethylpyrrolidin-1-yl, 3-pyrrolin-1-yl, 2,5-dimethyl-3-pyrrolin-1-yl, piperidino, 2-methylpiperidino, 2,6-dimethylpiperidino, piperazin-1-yl, morpholino, 1,2,3,4-tetrahydroquinolin-1-yl, 2-methyl-1,2,3,4-tetrahydroquinolin-1-yl, 2,2-dimethyl-1,2,3,4-tetrahydroquinolin-1-yl, 2,2-dimethyl-1,2-dihydroquinolin-1-yl or 6-fluoro-2,2-dimethyl-1,2-dihydroquinolin-1-yl,
R³ is hydrogen or C₁₋₄ alkyl,
R⁴ is hydrogen or C₁₋₄ alkyl, or
R³ and R⁴, together with the carbon atoms to which they are bonded, form cyclopentylidene or cyclohexylidene,
R⁵ is C₁₋₄ alkyl, C₂₋₄ alkenyl or benzyl, and
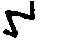 is a single bond of Anti form or of Syn form.

Among the compounds and mixtures of geometrical isomers of the formula (I), more preferred compounds are those
wherein,
R¹ and R² independently of one another represent C₁₋₄ alkyl, C₁₋₄ haloalkyl, cyclopropyl, cyclopentyl, cyclohexyl, C₂₋₄ alkenyl, C₂₋₄ haloalkenyl or C₃₋₄ alkynyl, or represent phenyl which may be substituted by methyl, fluoro, chloro or bromo, or
R¹ and R², together with the nitrogen atom to which they are bonded, form pyrrolidin-1-yl, 2,5-dimethylpyrrolidin-1-yl, 3-pyrrolin-1-yl, 2,5-dimethyl-3-pyrrolin-1-yl, piperidino, 2-methylpiperidino, 2,6-dimethylpiperidino, piperazin-1-yl, morpholino, 1,2,3,4-tetrahydroquinolin-1-yl, 2-methyl-1,2,3,4-tetrahydroquinolin-1-yl,2,2-dimethyl-1,2,3,4-tetrahydroquinolin-1-yl, 2,2-dimethyl-1,2-dihydroquinolin-1-yl or 6-fluoro-2,2-dimethyl-1,2-dihydroquinolin-1-yl,
R³ represents hydrogen or methyl,
R⁴ represents hydrogen, methyl or ethyl, or
R³ and R⁴, together with the carbon atoms to which they are bonded, form cyclopentylidene or cyclohexylidene,
R⁵ represents methyl, ethyl, allyl or benzyl, and
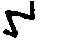 represents a single bond of Anti form or of Syn form.

Examples of the compounds of the formula (I) according to the invention include, in addtion to those mentioned in the below-described Examples, those shown in the following Tables 1, 2 and 3.

Table 1 shows the compounds according to the invention wherein R¹, R², R³ and R⁴ independently of one another represent a group.

Table 2 shows the compounds according to the invention wherein R¹ and R² independently of one another represent a group, and R³ and R⁴, together with the carbon atoms to which they are bonded, represent cyclopentylidene or cyclohexylidene.

Table 3 shows the compounds according to the invention wherein R¹ and R², together with the nitrogen atom to which they are bonded, represent a heterocyclic ring.

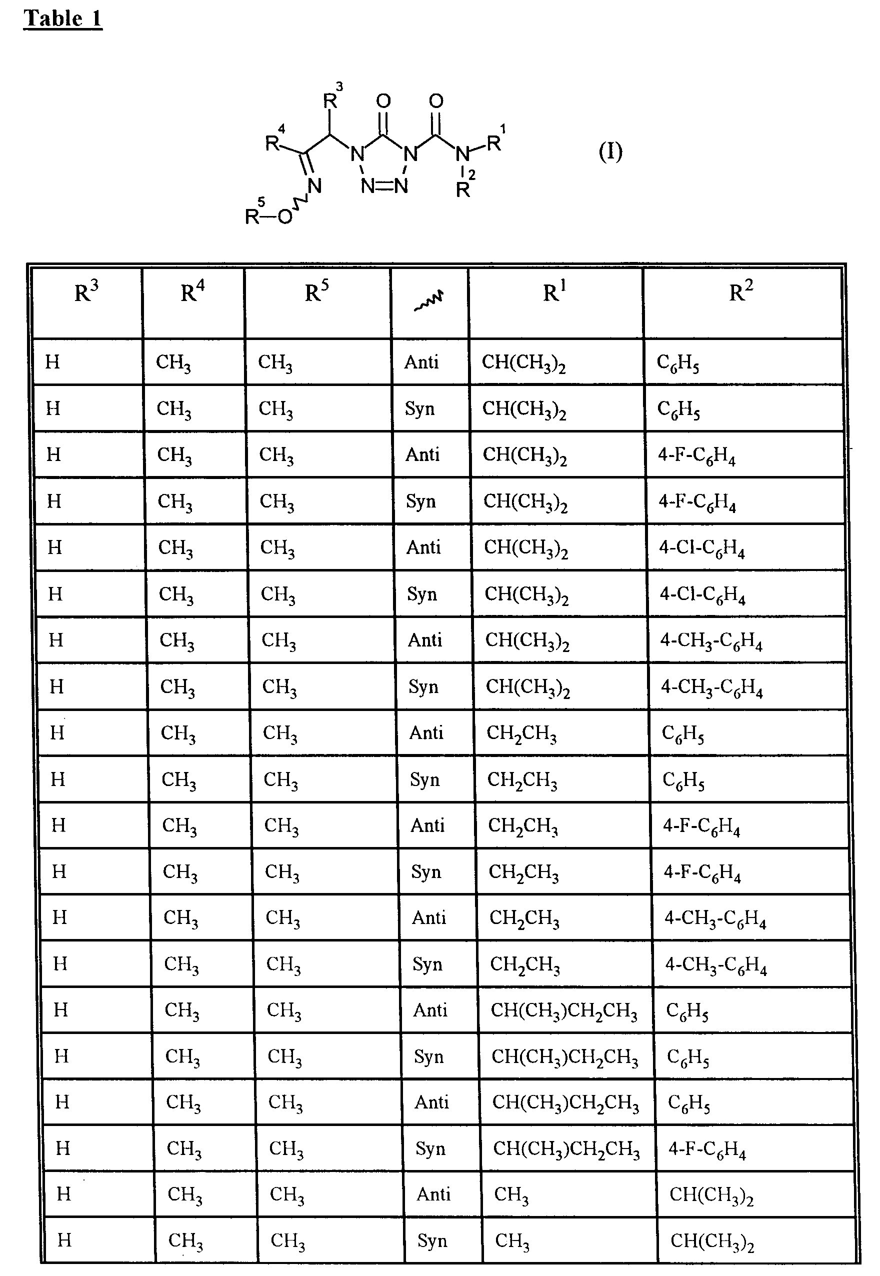

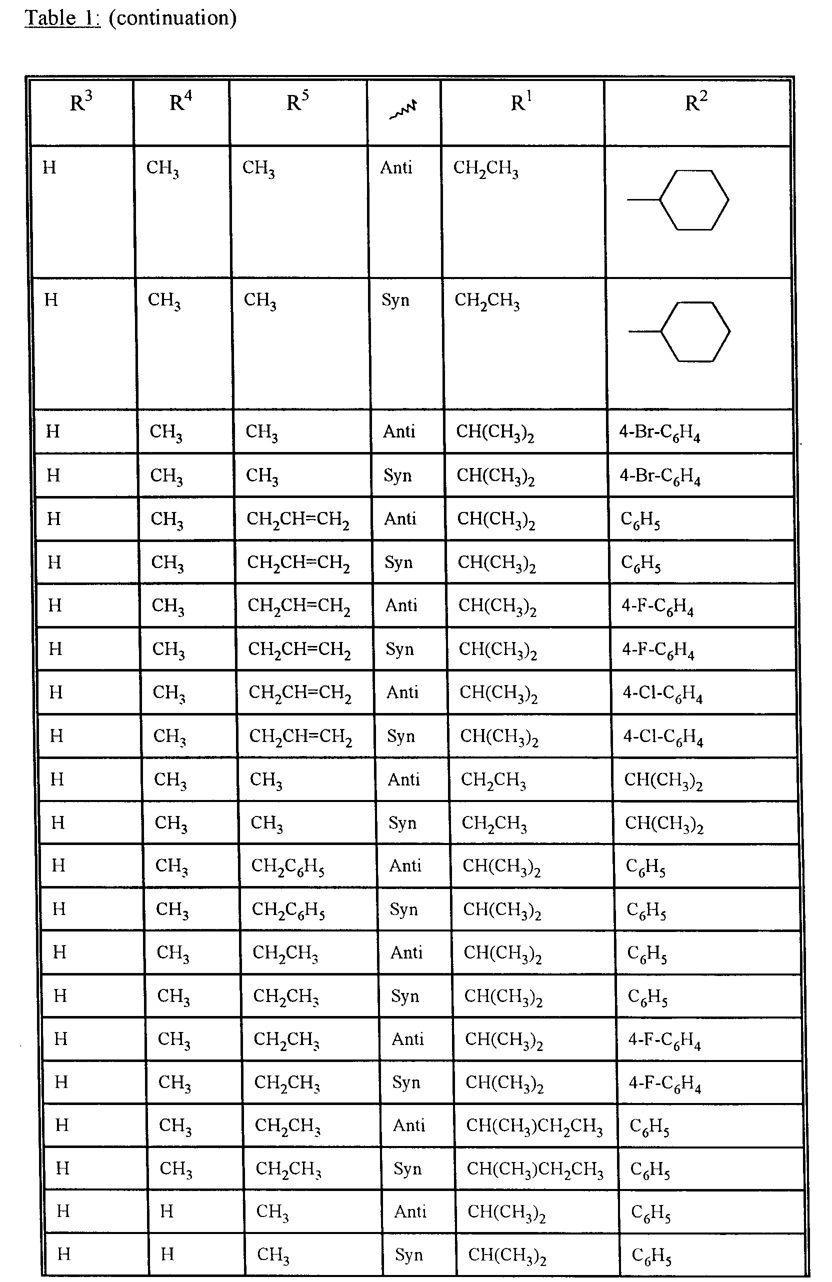

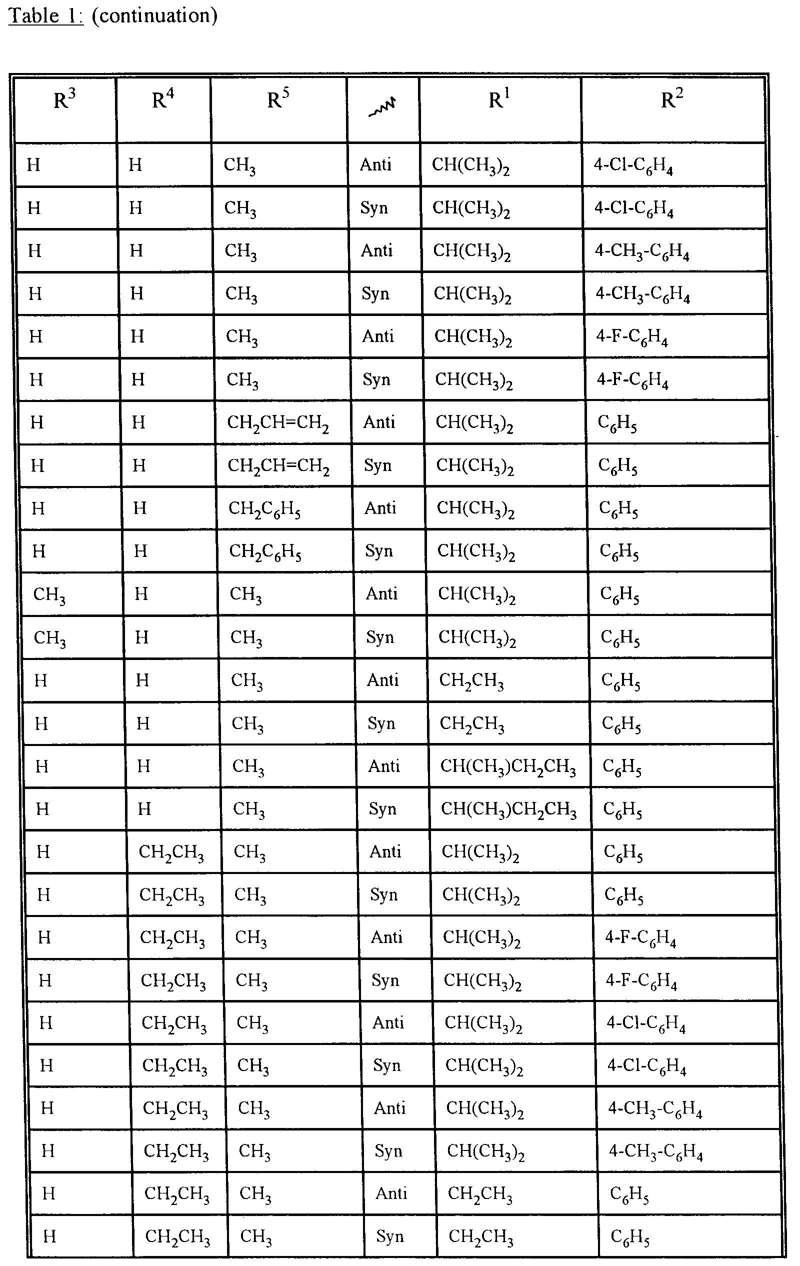

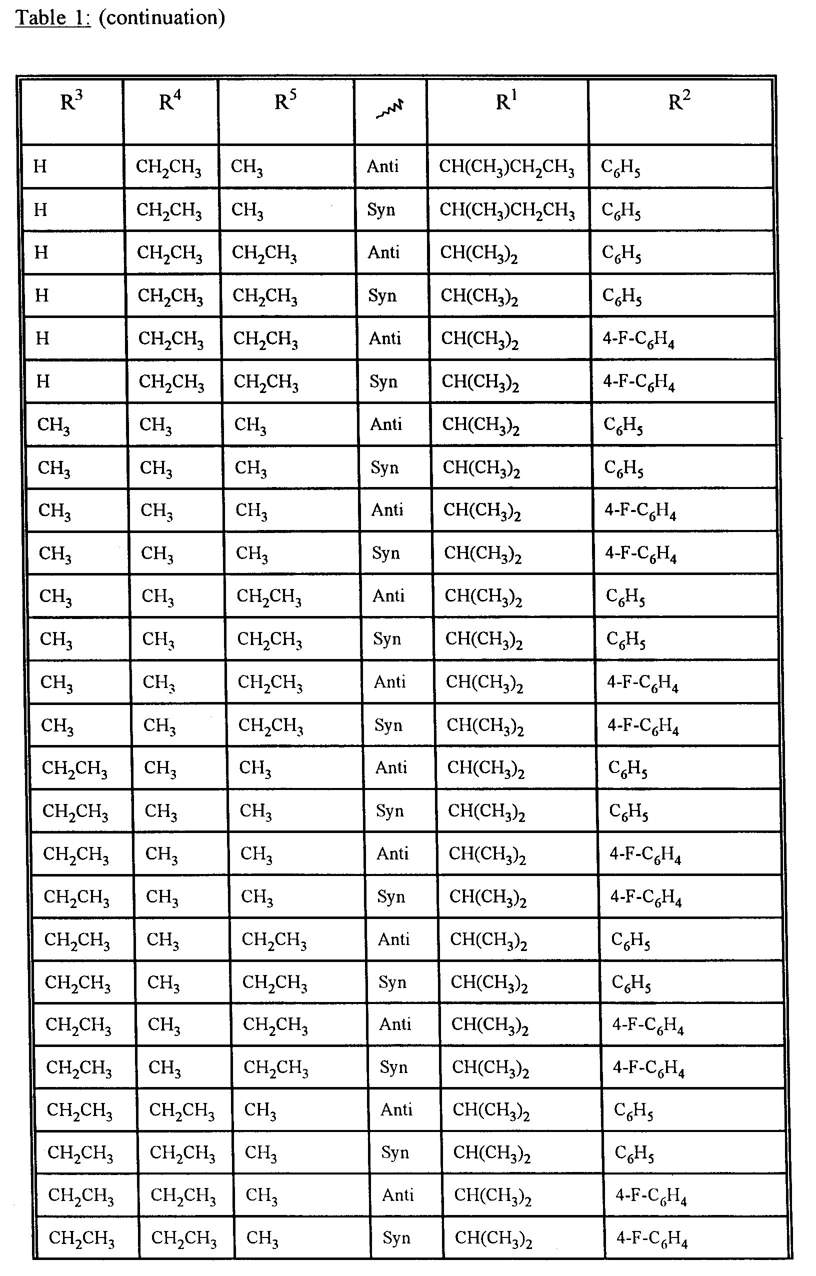

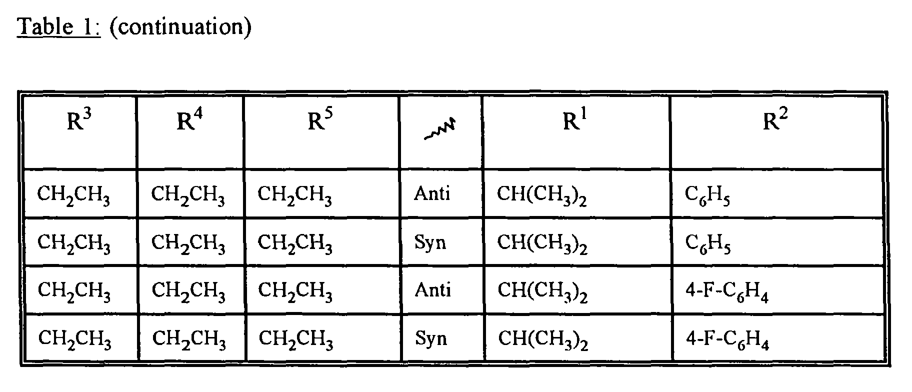

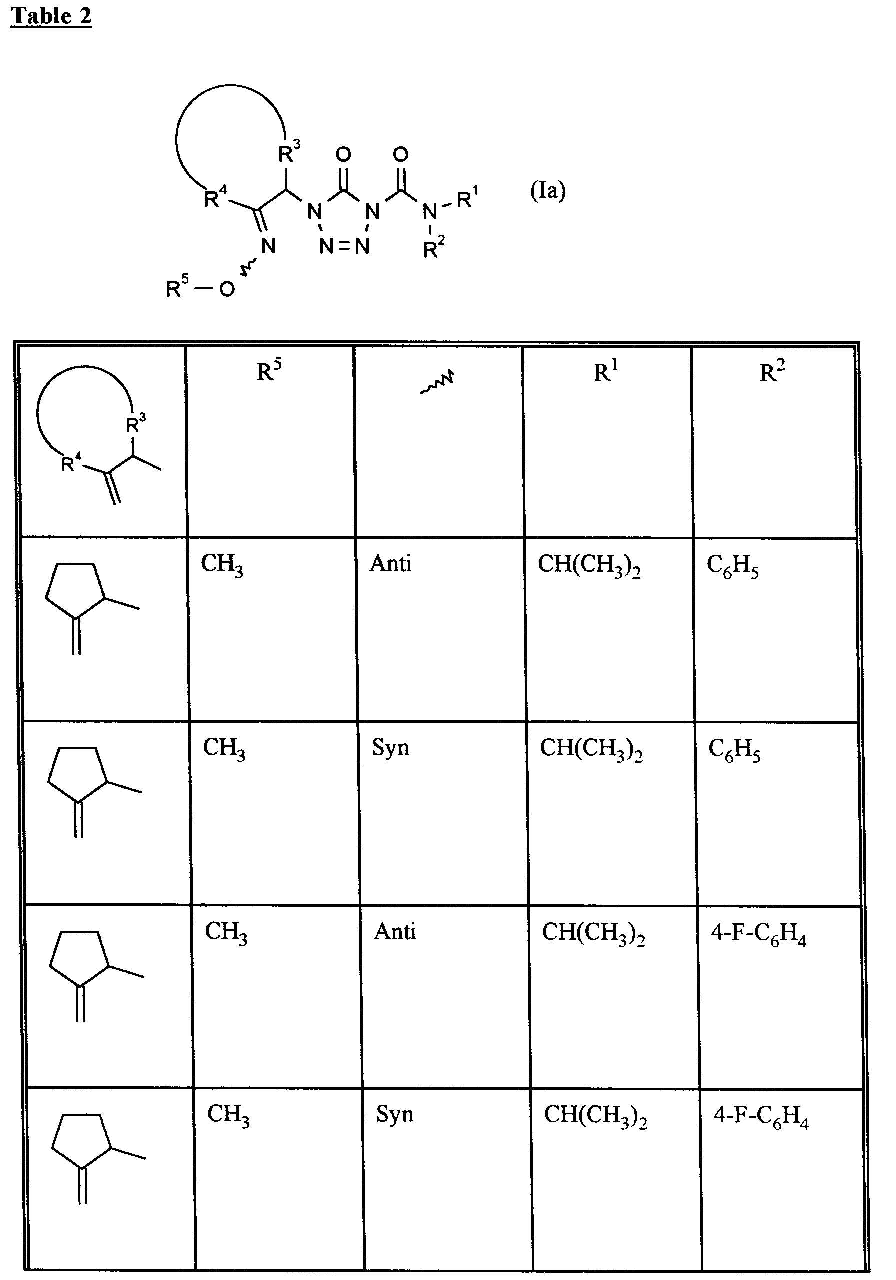

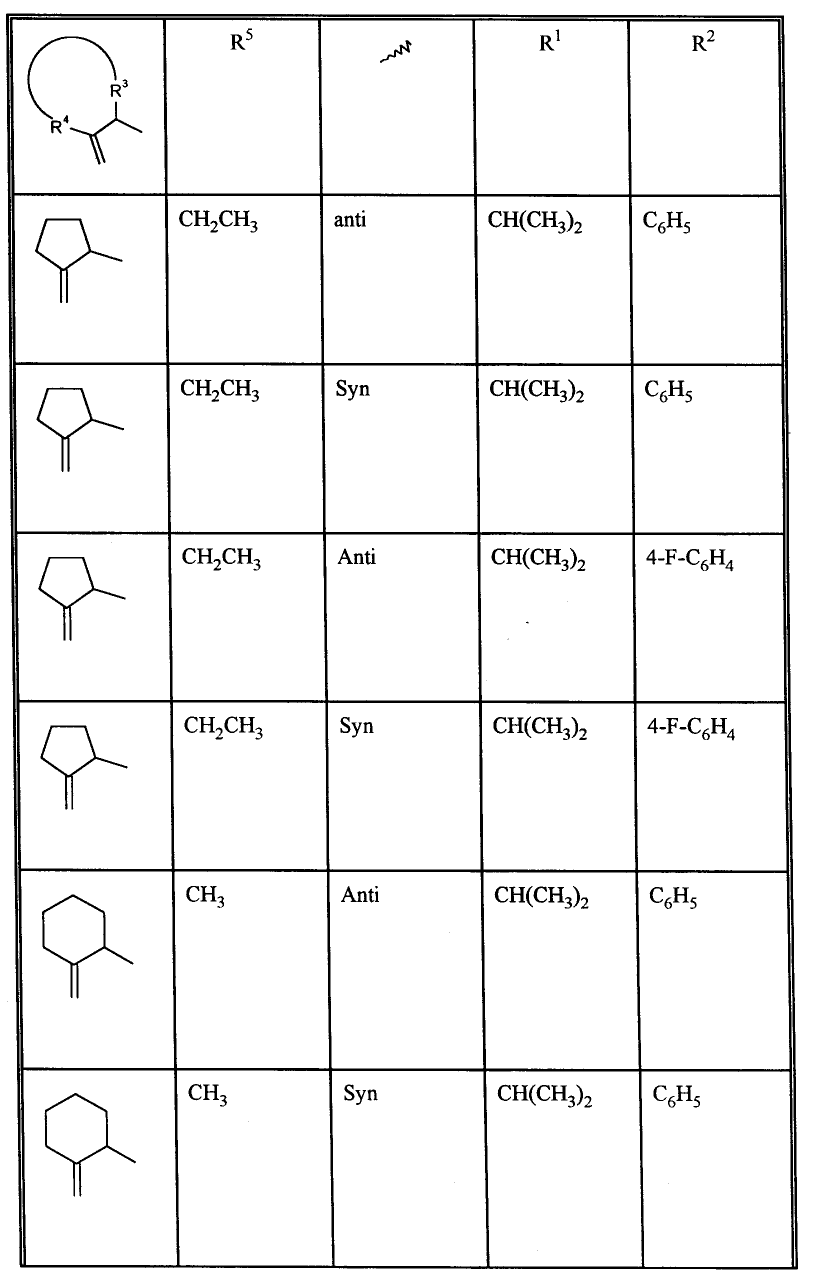

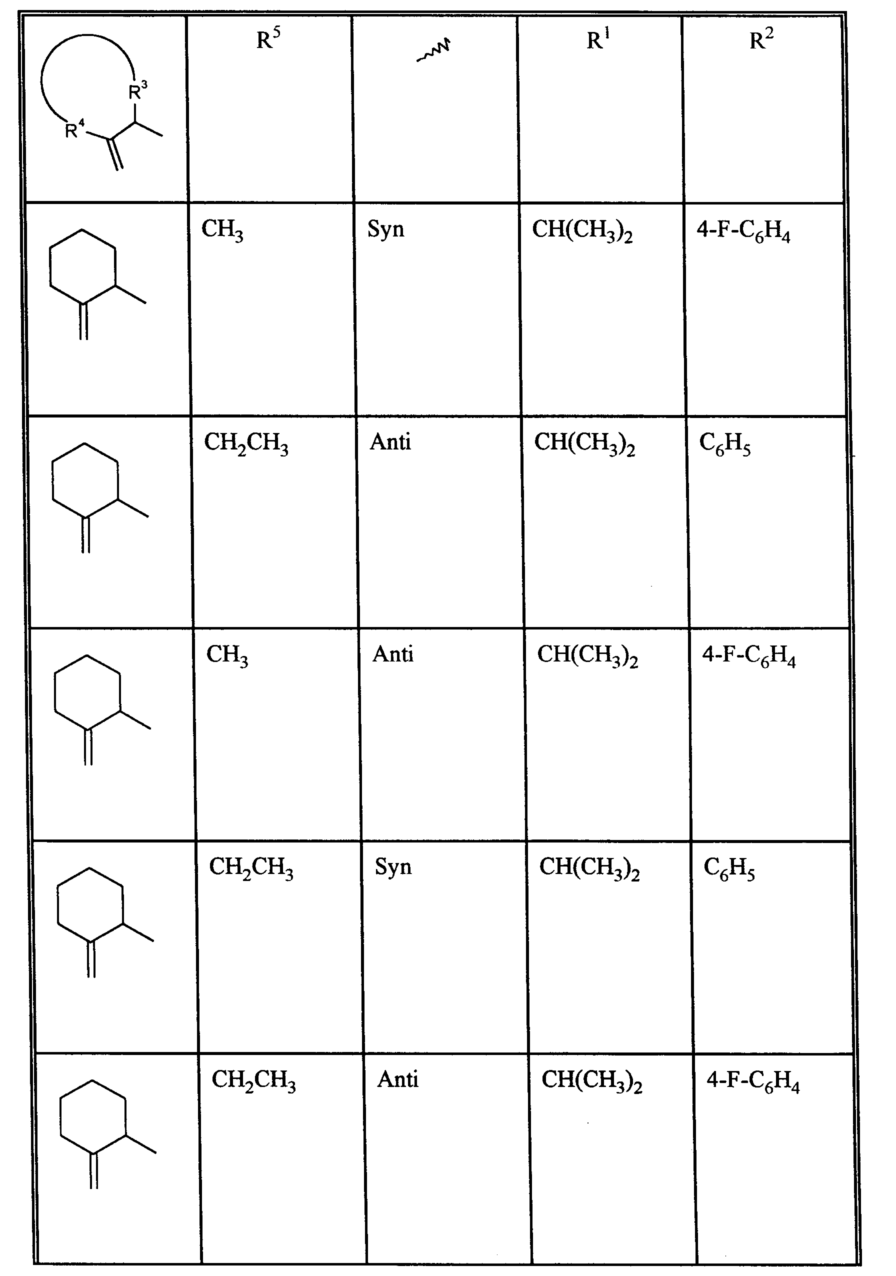

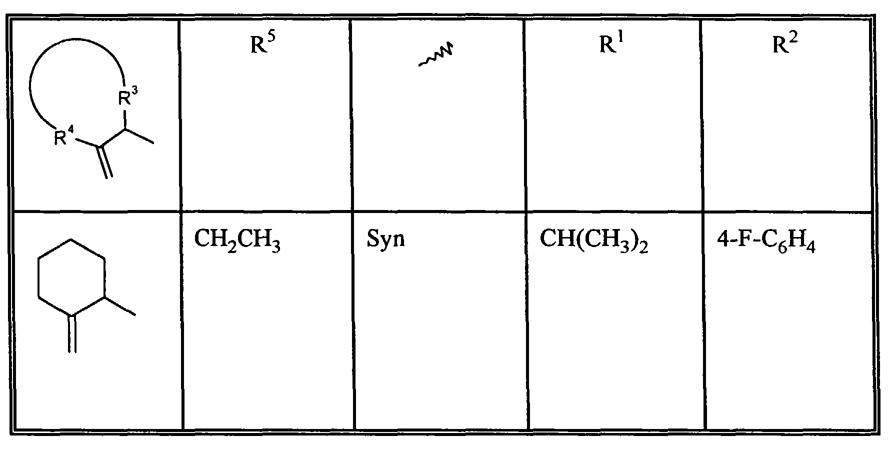

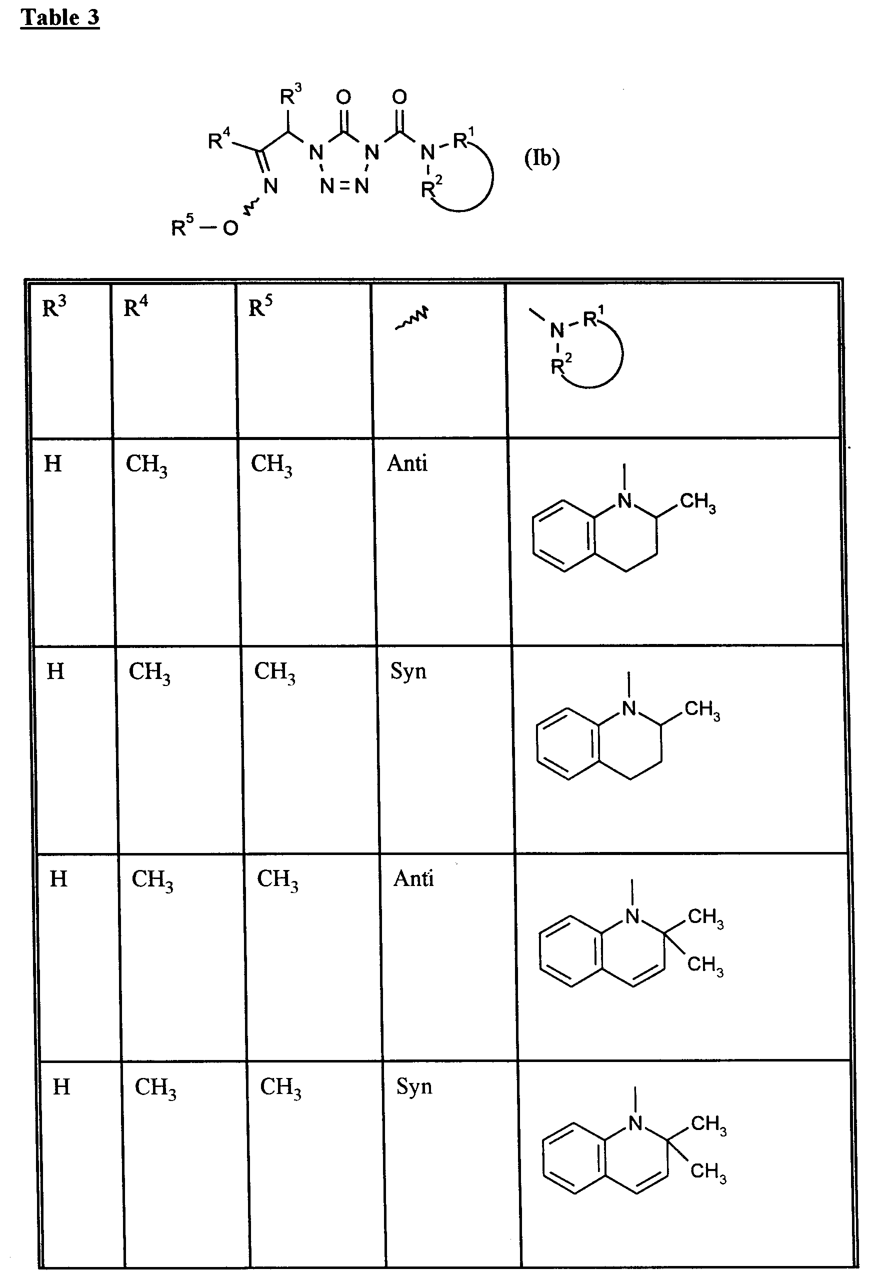

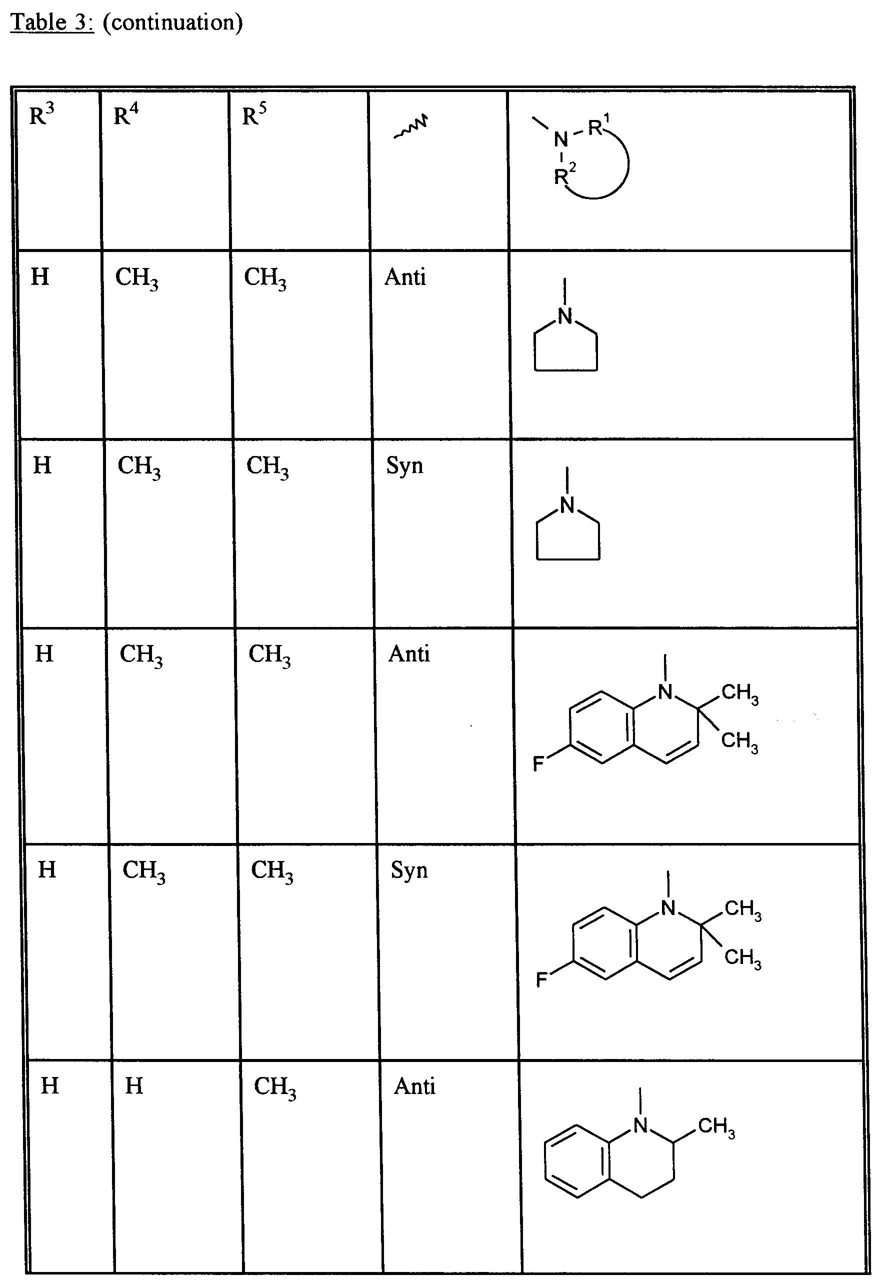

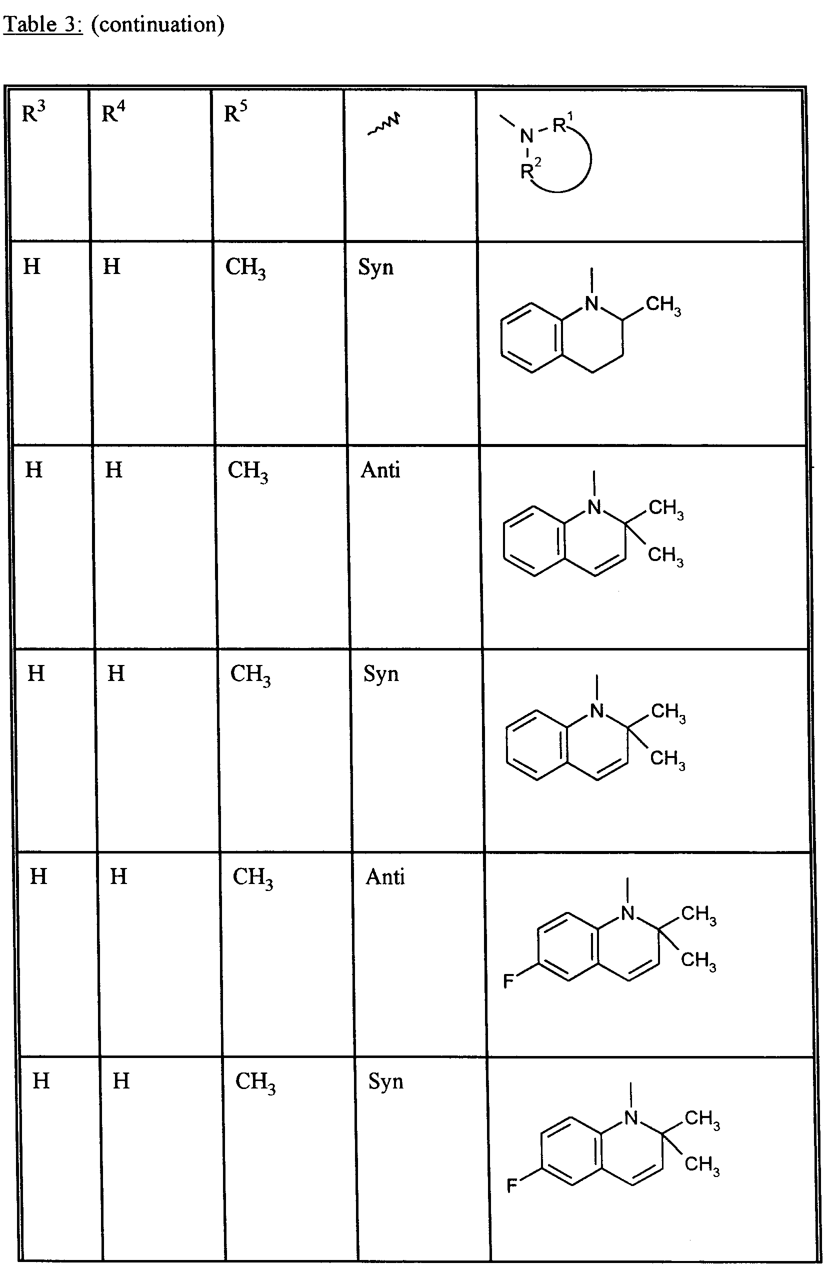

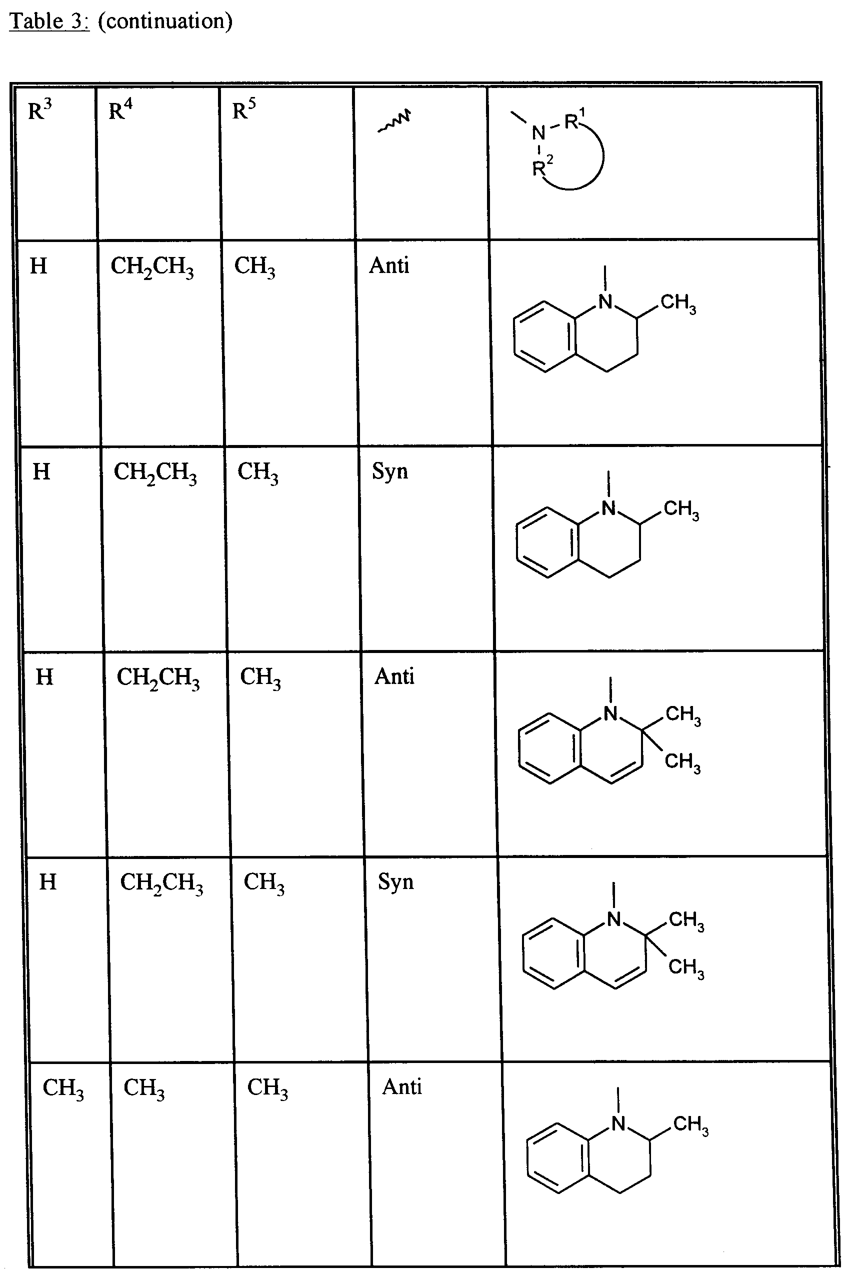

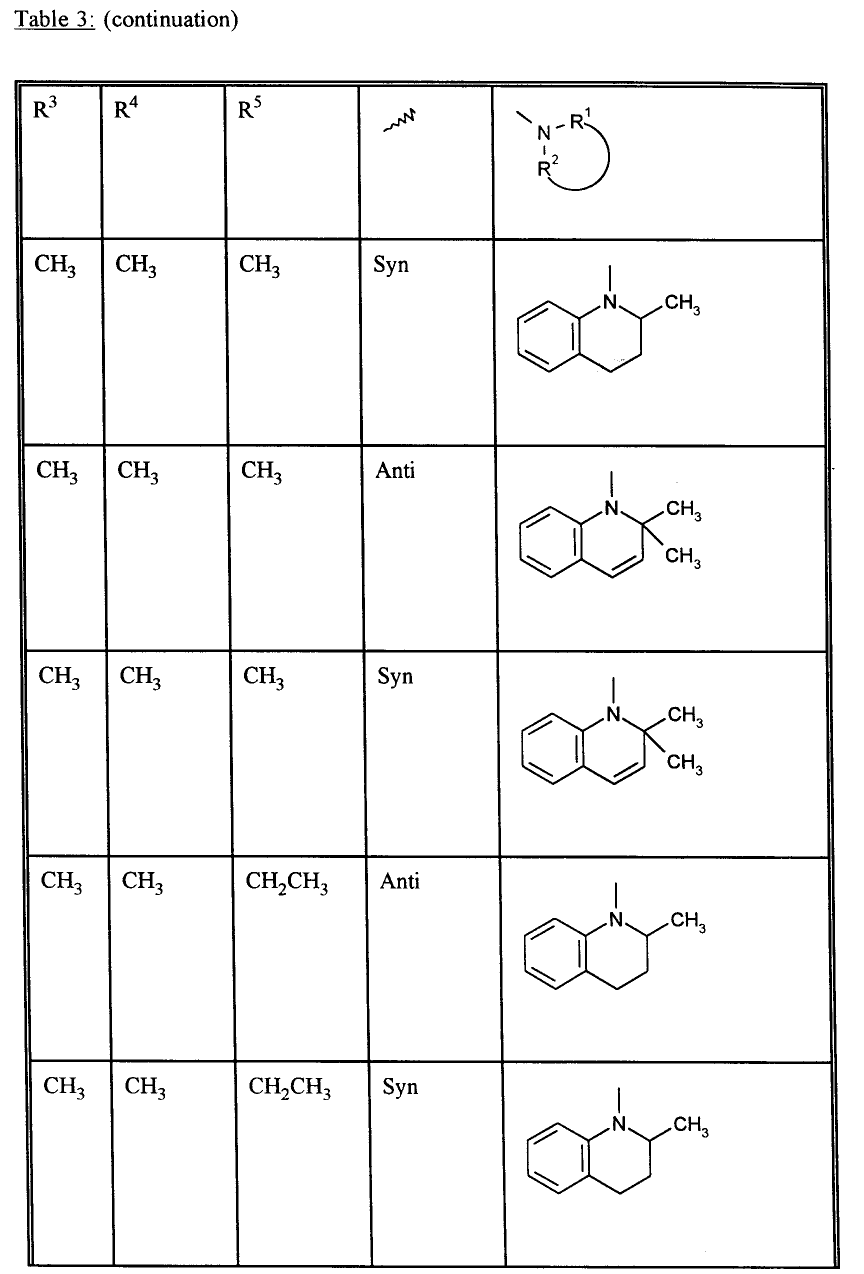

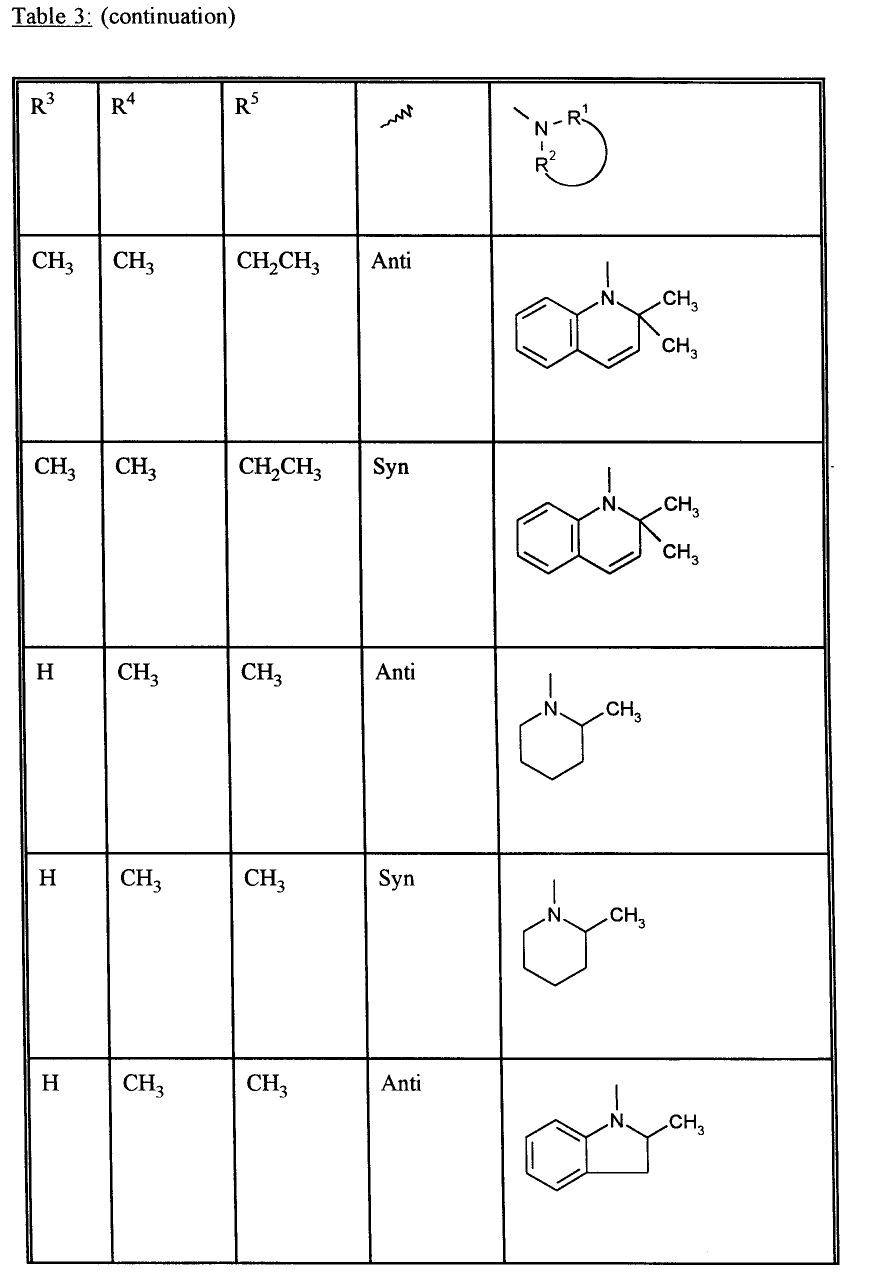

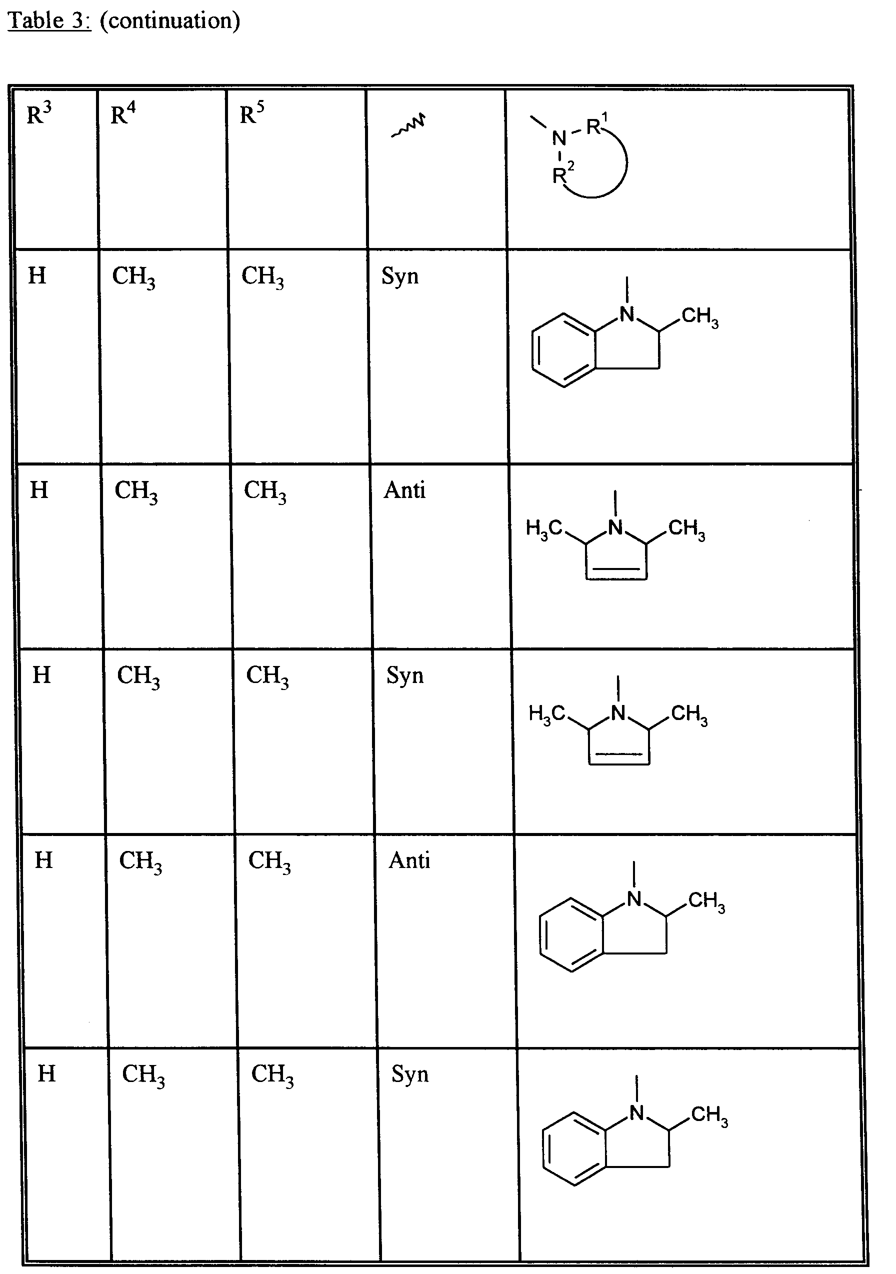

When in the above process (a), for example, 1-(2-methoxyiminopropyl)-5(4H)-tetrazolinone and N-(4-fluorophenyl)-N-isopropylcarbamoyl chloride are used as the starting materials, the course of the reaction can be represented by the following equation:

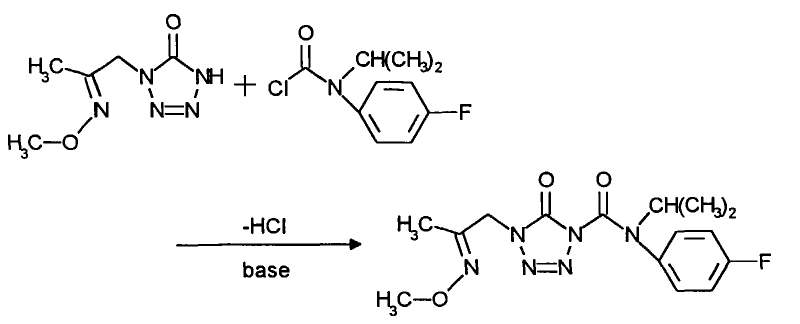

When in the process (b), for example, 1-(acetonyl)-4-(N-isopropyl-N-phenyl-carbamoyl)-5(4H)-tetrazolinone and O-methylhydroxylamine are used as starting materials, the course of the reaction can be represented by the following equation:

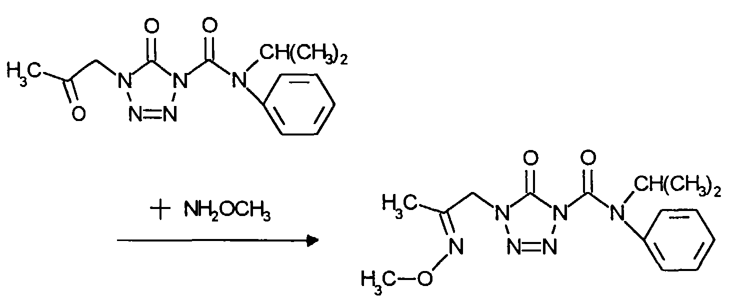

In the process (a) according to the invention, the compounds of formula (II) mean those based on the above definitions of R³, R⁴ and R⁵, and preferably those based on the above preferred definitions of R³, R⁴ and R⁵.

In the process (a), examples of the compounds of the formula (II) used as the starting materials include the following compounds:
1-(2-methoxyiminoethyl)-5(4H)-tetrazolinone,
1-(2-ethoxyiminoethyl)-5(4H)-tetrazolinone,
1-(2-allyloxyiminoethyl)-5(4H)-tetrazolinone,
1-(2-benzyloxyiminoethyl)-5(4H)-tetrazolinone,
1-(2-methoxyiminopropyl)-5(4H)-tetrazolinone,
1-(2-ethoxyiminopropyl)-5(4H)-tetrazolinone,
1-(2-allyloxyiminopropyl)-5(4H)-tetrazolinone,
1-(2-benzyloxyiminopropyl)-5(4H)-tetrazolinone,
1-(2-methoxyiminobutyl)-5(4H)-tetrazolinone,
1-(2-ethoxyiminobutyl)-5(4H)-tetrazolinone,
1-(1-methyl-2-methoxyiminopropyl)-5(4H)-tetrazolinone,
1-(2-ethoxyimino-1-methylpropyl)-5(4H)-tetrazolinone,
1-(1-ethyl-2-methoxyiminopropyl)-5(4H)-tetrazolinone,
1-(1-ethyl-2-ethoxyiminopropyl)-5(4H)-tetrazolinone,
1-(1-methyl-2-methoxyiminobutyl)-5(4H)-tetrazolinone,
1-(1-ethyl-2-ethoxyiminobutyl)-5(4H)-tetrazolinone,
1-(2-methoxyiminocyclopentyl)-5(4H)-tetrazolinone,
1-(2-methoxyiminocyclohexyl)-5(4H)-tetrazolinone,
1-(2-ethoxyiminocyclopentyl)-5(4H)-tetrazolinone,
1-(2-ethoxyiminocyclohexyl)-5(4H)-tetrazolinone, and so on.

The compounds of the formula (II) are novel and such compounds can be obtained by the following process:
(c)
   compounds of the formula (VI): 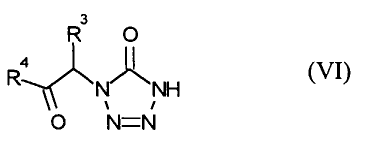 wherein
   - R³ and R⁴: have the above-mentioned meanings,
   are reacted with the compounds of the above formula (V),
   in the presence of inert solvents and, if appropriate, in the presence of an acid binder.

The above process (c) can be carried out under conditions similar to that of the below-described reaction of the process (b). In the process (c), examples of the compounds of the formula (VI) used as the starting materials are as follows:
1-formylmethyl-5(4H)-tetrazolinone,
1-acetonyl-5(4H)-tetrazolinone,
1-(2-oxobutyl)-5(4H)-tetrazolinone,
1-(1-methyl-2-oxopropyl)-5(4H)-tetrazolinone,
1-(1-ethyl-2-oxopropyl)-5(4H)-tetrazolinone,
1-(1-methyl-2-oxobutyl)-5(4H)-tetrazolinone,
1-(1-ethyl-2-oxobutyl)-5(4H)-tetrazolinone,
1-(2-oxo-cyclopentyl)-5(4H)-tetrazolinone,
1-(2-oxo-cyclohexyl)-5(4H)-tetrazolinone, etc.

In the process (c), the compounds of the formula (VI) as the starting materials are novel and such compounds can be obtained by the following processes:
(d)
   compounds of the formula (VII): 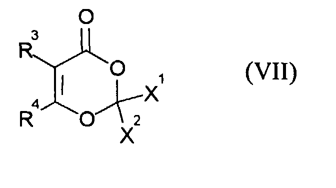 wherein
   - R³ and R⁴: have the above-mentioned meanings, and
   - X¹ and X²: independently of one another represent hydrogen or C₁₋₄ alkyl,
   are reacted with trimethylsilyl azide, if appropriate, in the presence of catalysts, and then are reacted with protic solvents,
   or
(e) in the case where R³ and R⁴ represent hydrogen:
   compounds of the formula (VIII): 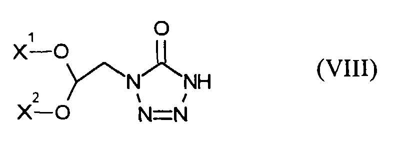 wherein
   - X¹ and X²: have the above-mentioned meanings,
   are hydrolyzed.

The above process (d) can be carried out by a procedure similar to those described in Journal of the Chemical Society, Perkin Transactions 1, 1995, 1101-1104 and by using the compounds of the above formula (VII) instead of the chlorides described in said publication. The compounds of the formula (VII) are well known in the field of organic chemistry and examples thereof include 2,2,6-trimethyl-4H-1,3-dioxin-4-one.

In the above process (e), the reaction per se is well known in the field of organic chemistry. The compounds of the formula (VIII) used as the starting materials are novel and such compounds can be produced by a process similar to that described in Japanese Patent Kokai Publication Hei 8-82258.

That is to say, 2,2-dialkoxyethylamine is reacted with carbon disulfide, and the reaction product is reacted with dimethyl sulfate to obtain methyl N-(2,2-dialkoxyethyl)- dithiocarbamate. Then, these compounds are reacted with sodium azide to obtain 1-(2,2-dialkoxyethyl)-(4H)-tetrazolin-5-thiones. These are further reacted with propylene oxide to obtain the objective compounds of the formula (VIII). Examples of the compounds of the formula (VIII) include 1-(2,2-dimethoxyethyl)-5(4H)-tetrazolinone.

In the above process (c), the compounds of the formula (V) used as the starting materials are the same as the starting materials in the below-described process (b).

In the process (a) according to the invention, the compounds of formula (III) mean those based on the above definitions of R¹, R² and hal, and preferably those based on the above prefered deffinitions of R¹ and R² , and preferably hal is chlorine or bromine.

In the above process (a), the compounds of the formula (III) used as the starting materials are well known in the field of organic chemistry and examples thereof include the following compounds:
diisopropylcarbamoyl chloride and bromide,
diethylcarbamoyl chloride and bromide,
dimethylcarbamoyl chloride and bromide,
N-methyl-N-ethylcarbamoyl chloride and bromide,
N-methyl-N-n-propylcarbamoyl chloride and bromide,
N-methyl-N-isopropylcarbamoyl chloride and bromide,
N-methyl-N-cyclopropylcarbamoyl chloride and bromide,
N-methyl-N-sec-butylcarbamoyl chloride and bromide,
N-methyl-N-cyclopentylcarbamoyl chloride and bromide,
N-methyl-N-cyclohexylcarbamoyl chloride and bromide,
N-methyl-N-phenylcarbamoyl chloride and bromide,
N-methyl-N-1-methyl-2-propenylcarbamoyl chloride and bromide,
N-ethyl-N-n-propylcarbamoyl chloride and bromide,
N-ethyl-N-isopropylcarbamoyl chloride and bromide,
N-ethyl-N-cyclopropylcarbamoyl chloride and bromide,
N-ethyl-N-sec-butylcarbamoyl chloride and bromide,
N-ethyl-N-cyclopentylcarbamoyl chloride and bromide,
N-ethyl-N-cyclohexylcarbamoyl chloride and bromide,
N-ethyl-N-phenylcarbamoyl chloride and bromide,
N-n-propyl-N-isopropylcarbamoyl chloride and bromide,
N-n-propyl-N-cyclopropylcarbamoyl chloride and bromide,
N-n-propyl-N-sec-butylcarbamoyl chloride and bromide,
N-n-propyl-N-cyclopentylcarbamoyl chloride and bromide,
N-n-propyl-N-cyclohexylcarbamoyl chloride and bromide,
N-isopropyl-N-cyclohexylcarbamoyl chloride and bromide,
N-isopropyl-N-phenylcarbamoyl chloride and bromide,
N-isopropyl-N-allylcarbamoyl chloride and bromide,
N-isopropyl-N-propargylcarbamoyl chloride and bromide,
N-isopropyl-N-(2-chloro-2-propenyl)carbamoyl chloride and bromide,
N-isopropyl-N-(2-methyl-2-propenyl)carbamoyl chloride and bromide,
N,N-diallylcarbamoyl chloride and bromide,
N,N-dipropargylcarbamoyl chloride and bromide,
N,N-di(2-chloroethyl)carbamoyl chloride and bromide,
morpholinocarbonyl chloride and bromide,
2-methylpiperidinocarbonyl chloride and bromide,
2,5-dimethylpyrrolidin-1-yl-carbonyl chloride and bromide,
2,6-dimethylpiperidinocarbonyl chloride and bromide,
2-methyl-1,2,3,4-tetrahydroquinolin-1-yl-carbonyl chloride and bromide,
2,2-dimethyl-1,2-dihydroquinolin-1-yl-carbonyl chloride and bromide,
pyrrolidin-1-ylcarbonyl chloride and bromide,
piperidinocarbonyl chloride and bromide,
2,5-dimethyl-3-pyrrolin-1-ylcarbonyl chloride and bromide, and so on.

In the above process (b), examples of the compounds of the formula (IV) used as the starting materials include the following compounds:
1-(formylmethyl)-4-(N-isopropyl-N-phenylcarbamoyl)-5(4H)-tetrazolinone,
1-(formylmethyl)-4-[N-isopropyl-N-(4-fluorophenyl)carbamoyl]-5(4H)-tetrazolinone,
1-(formylmethyl)-4-[N-isopropyl-N-(4-chlorophenyl)carbamoyl]-5(4H)-tetrazolinone,
1-(formylmethyl)-4-[N-isopropyl-N-(4-methylphenyl)carbamoyl]-5(4H)-tetrazolinone,
1-(formylmethyl)-4-(N-ethyl-N-phenylcarbamoyl)-5(4H)-tetrazolinone,
1-(formylmethyl)-4-(2-methyl-1,2,3,4-tetrahydroquinolin-1-ylcarbonyl)-5(4H)-tetrazolinone,
1-(formylmethyl)-4-(2,2-dimethyl-1,2-dihydroquinolin-1-ylcarbonyl)-5(4H)-tetrazolinone,
1-(formylmethyl)-4-(2,2-dimethyl-6-fluoro-1,2-dihydroquinolin-1-ylcarbonyl)-5(4H) -tetrazolinone,
1-(acetonyl)-4-(N-isopropyl-N-phenylcarbamoyl)-5(4H)-tetrazolinone,
1-(acetonyl)-4-[N-isopropyl-N-(4-fluorophenyl)carbamoyl]-5(4H)-tetrazolinone,
1-(acetonyl)-4-[N-isopropyl-N-(4-chlorophenyl)carbamoyl]-5(4H)-tetrazolinone,
1-(acetonyl)-4-[N-isopropyl-N-(4-methylphenyl)carbamoyl]-5(4H)-tetrazolinone,
1-(acetonyl)-4-(N-ethyl-N-phenylcarbamoyl)-5(4H)-tetrazolinone,
1-(acetonyl)-4-[N-ethyl-N-(4-fluorophenyl)carbamoyl]-5(4H)-tetrazolinone,
1-(acetonyl)-4-(N-sec-butyl-N-phenylcarbamoyl)-5(4H)-tetrazolinone,
1-(acetonyl)-4-[N-sec-butyl-N-(4-fluorophenyl)carbamoyl]-5(4H)-tetrazolinone,
1-(acetonyl)-4-(2-methyl-1,2,3,4-tetrahydroquinolin-1-ylcarbonyl)-5(4H)-tetrazolinone,
1-(acetonyl)-4-(2,2-dimethyl-1,2-dihydroquinolin-1-ylcarbonyl)-5(4H)-tetrazolinone,
1-(acetonyl)-4-(2,2-dimethyl-6-fluoro-1,2-dihydroquinolin-1-ylcarbonyl)-5(4H)-tetrazolinone,
1-(acetonyl)-4-(N-isopropyl-N-methylcarbamoyl)-5(4H)-tetrazolinone,
1-(2-oxobutyl)-4-(N-isopropyl-N-phenylcarbamoyl)-5(4H)-tetrazolinone,
1-(2-oxobutyl)-4-[N-isopropyl-N-(4-fluorophenyl)carbamoyl]-5(4H)-tetrazolinone,
1-(2-oxobutyl)-4-[N-isopropyl-N-(4-chlorophenyl)carbamoyl]-5(4H)-tetrazolinone,
1-(2-oxobutyl)-4-[N-isopropyl-N-(4-methylphenyl)carbamoyl]-5(4H)- tetrazolinone,
1-(2-oxobutyl)-4-(N-ethyl-N-phenylcarbamoyl)-5(4H)-tetrazolinone,
1-(2-oxobutyl)-4-(2-methyl-1,2,3,4-tetrahydroquinolin-1-ylcarbonyl)-5(4H)-tetrazolinone,
1-(2-oxobutyl)-4-(2,2-dimethyl-1,2-dihydroquinolin-1-ylcarbonyl)-5(4H)-tetrazolinone,
1-(2-oxobutyl)-4-(2,2-dimethyl-6-fluoro-1,2-dihydroquinolin-1-ylcarbonyl)-5(4H)-tetrazolinone,
1-(1-methyl-2-oxopropyl)-4-(N-isopropyl-N-phenylcarbamoyl)-5(4H)-tetrazolinone,
1-(1-methyl-2-oxopropyl)-4-[N-isopropyl-N-(4-fluorophenyl)carbamoyl]-5(4H)-tetrazolinone,
1-(1-methyl-2-oxopropyl)-4-(2-methyl-1,2,3,4-tetrahydroquinolin-1-ylcarbonyl)-5(4 H)- tetrazolinone,
1-(1-methyl-2-oxopropyl)-4-(2,2-dimethyl-1,2-dihydroquinolin-1-ylcarbonyl)-5(4H)-tetrazolinone,
1-(1-ethyl-2-oxopropyl)-4-(N-isopropyl-N-phenylcarbamoyl)-5(4H)-tetrazolinone,
1-(1-ethyl-2-oxopropyl)-4-[N-isopropyl-N-(4-fluorophenyl)carbamoyl]-5(4H)-tetrazolinone,
1-(1-methyl-2-oxobutyl)-4-(N-isopropyl-N-phenylcarbamoyl)-5(4H)-tetrazolinone,
1-(1-methyl-2-oxobutyl)-4-[N-isopropyl-N-(4-fluorophenyl)carbamoyl]-5(4H)-tetrazolinone,
1-(1-ethyl-2-oxobutyl)-4-(N-isopropyl-N-phenylcarbamoyl)-5(4H)-tetrazolinone,
1-(1-ethyl-2-oxobutyl)-4-[N-isopropyl-N-(4-fluorophenyl)carbamoyl]-5(4H)-tetrazolinone,
1-(2-oxo-cyclopentyl)-4-(N-isopropyl-N-phenylcarbamoyl)-5(4H)-tetrazolinone,
1-(2-oxo-cyclopentyl)-4-[N-isopropyl-N-(4-fluorophenyl)carbamoyl]-5(4H)-tetrazolinone,
1-(2-oxo-cyclohexyl)-4-(N-isopropyl-N-phenylcarbamoyl)-5(4H)-tetrazolinone,
1-(2-oxo-cyclohexyl)-4-[N-isopropyl-N-(4-fluorophenyl)carbamoyl]-5(4H)-tetrazolinone, and so on.

The compounds of the formula (IV) are novel and such compounds can be obtained, for example, by the following processes:
(f)
   the compounds of the above formula (VI) are reacted with the compounds of the above formula (III) under conditions similar to that of the process (a),
   or
(g) in the case where R³ and R⁴ represent hydrogen:
   compounds of the formula (IX): 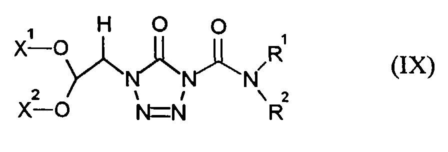 wherein
   - R¹, R², X¹ and X²: have the above-mentioned meanings,
   are hydrolyzed.

The compounds of the formula (IX) are novel and include, for example, the following compounds:
1-(2,2-dimethoxyethyl)-4-(N-isopropyl-N-phenylcarbamoyl)-5(4H)-tetrazolinone,
1-(2,2-dimethoxyethyl)-4-(N-isopropyl-N-(4-fluorophenyl)carbamoyl)-5(4H)-tetrazolinone,
1-(2,2-dimethoxyethyl)-4-(N-isopropyl-N-(4-chlorophenyl)carbamoyl)-5(4H)-tetrazolinone,
1-(2,2-dimethoxyethyl)-4-(N-isopropyl-N-(4-methylphenyl)carbamoyl)-5(4H)-tetrazolinone,
1-(2,2-dimethoxyethyl)-4-(N-ethyl-N-phenylcarbamoyl)-5(4H)-tetrazolinone,
1-(2,2-dimethoxyethyl)-4-(2-methyl-1,2,3,4-tetrahydroquinolin-1-ylcarbonyl)-5(4H)-tetrazolinone,
1-(2,2-dimethoxyethyl)-4-(2,2-methyl-1,2-dihydroquinolin-1-ylcarbonyl)-5(4H)-tetrazolinone,
1-(2,2-dimethoxyethyl)-4-(2,2-methyl-6-fluoro-1,2-dihydroquinolin-1-ylcarbonyl)-5(4H)-tetrazolinone, etc.

The compounds of the formula (IX) can be obtained, for example, by the following process:
(h)
   the compounds of the above formula (VIII) are reacted with the compounds of the above formula (III) under conditions similar to that of the process (a).

In the process (b), the compounds of the formula (V) are well known in the field of organic chemistry and examples thereof include the following compounds:
O-methylhydroxylamine and the hydrochloric acid salt thereof,
O-ethylhydroxylamine and the hydrochloric acid salt thereof,
O-allylhydroxylamine and the hydrochloric acid salt thereof,
O-benzylhydroxylamine and the hydrochloric acid salt thereof, etc.

The compounds of the formulae (II), (IV), (VI), (VIII) and (IX) which are starting materials for the production of the compounds of the formula (I) according to the invention are novel, and such compounds are generally represented by the following formula (X): 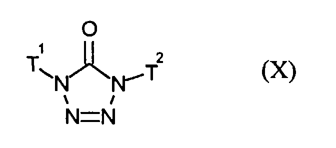 wherein
T¹ is hydrogen, 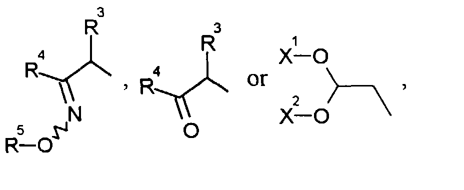
T² is hydrogen or 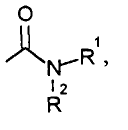 with the proviso that when T¹ is 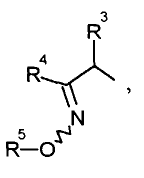 then
T² is hydrogen,
R¹ and R², independently of one another, are C₁₋₆ alkyl, C₁₋₆ haloalkyl, C₃₋₈ cycloalkyl, C₂₋₆ alkenyl, C₂₋₆ haloalkenyl, C₃₋₆ alkynyl or optionally substituted phenyl, or
R¹ and R², together with the nitrogen atom to which they are bonded, form a 5-membered or 6-membered heterocyclic ring which may be benzo-fused or which may be substituted by halogen or C₁₋₄ alkyl,
R³ is hydrogen or C₁₋₆ alkyl,
R⁴ is hydrogen or C₁₋₆ alkyl, or
R³ and R⁴, together with the carbon atoms to which they are bonded, form cyclopentylidene or cyclohexylidene,
R⁵ is C₁₋₆ alkyl, C₃₋₆ alkenyl or benzyl,
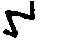 is a single bond of Anti form or of Syn form, and
X¹ and X² independently of one another are hydrogen or C₁₋₄ alkyl.

Preferred compounds of the formula (X) are compounds and mixtures of geometrical isomers,
wherein
T¹ is hydrogen, 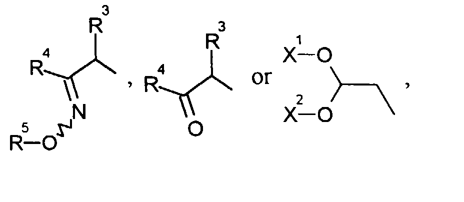
T² is hydrogen or 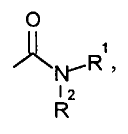 with the proviso that when T¹ is 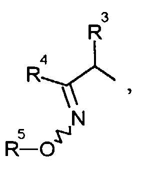 then
T² is hydrogen,
R¹ and R², independently of one another, are C₁₋₄ alkyl, C₁₋₄ haloalkyl, cyclopropyl, cyclopentyl, cyclohexyl, C₂₋₄ alkenyl, C₂₋₄ haloalkenyl or C₃₋₄ alkynyl, or are phenyl which may be substituted by halogen or C₁₋₄ alkyl, or
R¹ and R², together with the nitrogen atom to which they are bonded, form pyrrolidin-1-yl, 2,5-dimethylpyrrolidin-1-yl, 3-pyrrolin-1-yl, 2,5-dimethyl-3-pyrrolin-1-yl, piperidino, 2-methylpiperidino, 2,6-dimethylpiperidino, piperazin-1-yl, morpholino, 1,2,3,4-tetrahydroquinolin-1-yl, 2-methyl-1,2,3,4-tetrahydroquinolin-1-yl, 2,2-dimethyl-1,2,3,4-tetrahydroquinolin-1-yl, 2,2-dimethyl-1,2-dihydroquinolin-1-yl or 6-fluoro-2,2-dimethyl-1,2-dihydroquinolin-1-yl,
R³ is hydrogen or C₁₋₄ alkyl,
R⁴ is hydrogen or C₁₋₄ alkyl, or
R³ and R⁴, together with the carbon atoms to which they are bonded, form cyclopentylidene or cyclohexylidene,
R⁵ is C₁₋₄ alkyl, C₂₋₄ alkenyl or benzyl,
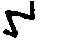 is a single bond of Anti form or of Syn form, and
X¹ and X² independently of one another are hydrogen or C₁₋₃ alkyl.

More preferred compounds of the formula (X) are compounds and mixtures of geometrical isomers, wherein
T¹ is hydrogen, 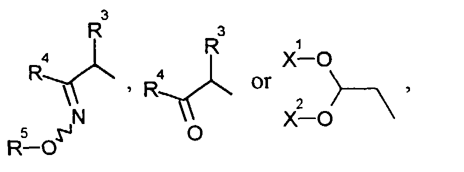
T² is hydrogen or 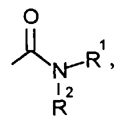 with the proviso that when
T¹ represents 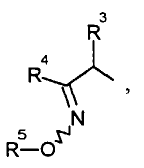 then T² is hydrogen,
R¹ and R², independently of one another, are C₁₋₄ alkyl, C₁₋₄ haloalkyl, cyclopropyl, cyclopentyl, cyclohexyl, C₂₋₄ alkenyl, C₂₋₄ haloalkenyl or C₃₋₄ alkynyl, or represent phenyl which may be substituted by methyl, fluoro, chloro or bromo, or
R¹ and R², together with the nitrogen atom to which they are bonded, form pyrrolidin-1-yl, 2,5-dimethylpyrrolidin-1-yl, 3-pyrrolin-1-yl, 2,5-dimethyl-3-pyrrolin-1-yl, piperidino, 2-methylpiperidino, 2,6-dimethylpiperidino, piperazin-1-yl, morpholino, 1,2,3,4-tetrahydroquinolin-1-yl, 2-methyl-1,2,3,4-tetrahydroquinolin-1-yl, 2,2-dimethyl-1,2,3,4-tetrahydroquinolin-1-yl, 2,2-dimethyl-1,2-dihydroquinolin-1-yl or 6-fluoro-2,2-dimethyl-1,2-dihydroquinolin-1-yl,
R³ is hydrogen or methyl,
R⁴ is hydrogen, methyl or ethyl, or
R³ and R⁴, together with the carbon atoms to which they are bonded, form cyclopentylidene or cyclohexylidene,
R⁵ is methyl, ethyl, allyl or benzyl,
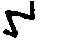 is a single bond of Anti form or of Syn form, and
X¹ and X² independently of one another are hydrogen or methyl.

The above process (a) can be carried out by a procedure similar to that described for the production of tetrazolinones in Japanese Patent Kokai Publication Hei 7-118246.

The reaction of the above process (a) can be carried out in an appropriate diluent. As usable diluents, there may be mentioned aliphatic, alicyclic or aromatic hydrocarbons (which may be optionally chlorinated), such as pentane, hexane, cyclohexane, petroleum ether, ligroin, benzene, toluene, xylene, dichloromethane, chloroform, carbon tetrachloride, 1,2-dichloroethane, chlorobenzene and dichlorobenzene; ethers such as diethyl ether, methyl ethyl ether, diisopropyl ether, dibutyl ether, dioxane, dimethoxyethane (DME), tetrahydrofuran (THF) and diethylene glycol dimethyl ether (DGM); nitriles such as acetonitrile and propionitrile; acid amides such as dimethylformamide (DMF), dimethylacetamide (DMA), N-methylpyrrolidone, 1,3-dimethyl-2-imidazolidinone and hexamethylphosphoric triamide (HMPA); sulfones and sulfoxides such as dimethyl sulfoxide (DMSO) and sulforan; and bases such as pyridine.

The reaction of the process (a) can be carried out in the presence of an acid binding agent. Examples of usable acid binding agents are as follows: inorganic bases, for example, hydroxides, carbonates and bicarbonates of alkali metals or alkaline earth metals, such as sodium hydrogencarbonate, potassium hydrogen-carbonate, sodium carbonate, potassium carbonate, lithium hydroxide, sodium hydroxide, potassium hydroxide, calcium hydroxide, sodium methoxide, potassium methoxide and potassium tert-butoxide; inorganic alkali metal amides, such as lithium amide, sodium amide and potassium amide; organic bases, for example, tertiary amines, dialkylaminoanilines and pyridines, such as trimethylamine, triethylamine, 1,1,4,4-tetramethylethylenediamine (TMEDA), N,N-dimethylaniline, N,N-diethylaniline, pyridine, 4-dimethylaminopyridine (DMAP), 1,4-diazabicyclo[2,2,2]octane (DABCO) and 1,8-diazabicyclo [5,4,0]undec-7-ene (DBU); organic lithium compounds, such as methyl lithium, n-butyl lithium, sec-butyl lithium, tert-butyl lithium, phenyllithium, dimethyl copper lithium, lithium diisopropylamide, lithium cyclohexylisopropylamide, lithium dicyclohexylamide, n-butyl lithium-DABCO, n-butyl lithium-DBU and n-butyl lithium-TMEDA.

The reaction of the process (a) can be conducted at a temperature within a substantially broad range, but it is generally possible to employ a reaction temperature of about -30 to about 200°C, preferably about -20 to about 130°C. Further, the reaction should preferably be conducted under normal pressure but it may optionally be operated under elevated or reduced pressure.

For carrying out the process (a), for instance, 1 mole of the compound of the formula (II) can be reacted with 1 to 1.5 moles of the compound of the formula (III) in a diluent such as toluene and in the presence of 1 to 1.5 moles of an acid binding agent to thereby obtain the objective compound of the formula (I).

Further, the reaction of the above process (b) can be carried out in an appropriate diluent. Examples of usable diluents are: water; aliphatic, alicyclic or aromatic hydrocarbons (which may be optionally chlorinated), such as pentane, hexane, cyclohexane, petroleum ether, ligroin, benzene, toluene, xylene, dichloromethane, chloroform, carbon tetrachloride, 1,2-dichloroethane, chlorobenzene and dichlorobenzene; ethers such as ethyl ether, methyl ethyl ether, isopropyl ether, butyl ether, dioxane, dimethoxyethane (DME), tetrahydrofuran (THF) and diethylene glycol dimethyl ether (DGM); nitriles such as acetonitrile, propionitrile and acrylonitrile; alcohols such methanol, ethanol, isopropanol, butanol and ethylene glycol; esters such as ethyl acetate and amyl acetate; acid amides such as dimethylformamide (DMF), dimethylacetamide (DMA), N-methylpyrrolidone, 1,3-dimethyl-2-imidazolidinone and hexamethylphosphoric triamide (HMPA); and sulfones and sulfoxides such as dimethyl sulfoxide (DMSO) and sulfolan.

The reaction of the process (b) can be carried out in the presence of acidic material as a catalyst. Examples of usable acidic materials are: mineral acids such as hydrochloric acid, sulfuric acid, nitric acid, hydrobromic acid, sodium hydrogensulfite; organic acids such as formic acid, acetic acid, trifluoroacetic acid, propionic acid, methanesulfonic acid, benzenesulfonic acid, p-toluenesulfonic acid; organic amine hydrochloric acid salts such as a pyridine-hydrochloric acid salt, triethylamine-hydrochloric acid salt; etc.

The reaction of the process (b) can be conducted at a temperature within a substantially broad range, but it is generally possible to employ a reaction-temperature of about -10 to about 200°C, preferably about 0 to about 120°C. Further, the reaction should preferably be conducted under normal pressure but it may optionally be operated under elevated or reduced pressure.

For carrying out the process (b), for instance, 1 mole of the compound of the formula (IV) can be reacted with 1.0 to 2.0 moles of the compound of the formula (V) in a diluent such as tetrahydrofuran and in the presence of sodium acetate to thereby obtain the objective compound of formula (I).

The active compounds of formula (I) according to the invention can be used as herbicides.

By weeds, in the broadest sense, there are to be understood all plants which grow in locations where they are undesired. Whether the substances according to the invention act as total or selective herbicides depends essentially on the amount used.

The active compounds according to the invention can be used, for example, in connection with the following plants:

Dicotyledon weeds of the genera: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver and Centaurea.

Dicotyledon cultures of the genera: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis and Cucurbita.

Monocotyledon weeds of the genera: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus and Apera.

Monocotyledon cultures of the genera: Oryza, Zea, Triticum, Hordeum, Avena, Secale, Sorghum, Panicum, Saccharum, Ananas, Asparagus and Allium.

However, the use of the active compounds according to the invention is in no way restricted to these genera, but also extends in the same manner to other plants.

The compounds are suitable, depending on the concentration, for the total combating of weeds, for example on industrial terrain and rail tracks, and on paths and squares with or without tree plantings. Equally, the compounds can be employed for combating weeds in perennial cultures, for example afforestations, decorative tree plantings, orchards, vineyards, citrus groves, nut orchards, banana plantations, coffee plantations, tea plantations, rubber plantations, oil palm plantations, cocoa plantations, soft fruit plantings and hopfields, and for the selective combating of weeds in annual cultures.

The active compounds can be converted into the customary formulations, such as solutions, emulsions, wettable powders, suspensions, powders, foams, pastes, granules, tablets, aerosols, natural and synthetic materials impregnated with active compound, very fine capsules in polymeric substances, coating compositions for use on seed, and formulations used with burning equipment, such as fumigating cartridges, fumigating cans and fumigating coils, as well as ULV cold mist and warm mist formulations.

These formulations may be produced in known manner, for example by mixing the active compounds with extenders, that is to say liquid or liquefied gaseous or solid diluents or carriers, optionally with the use of surface-active agents, that is to say emulsifying agents and/or dispersing agents and/or foam-forming agents. In the case of the use of water as an extender, organic solvents can, for example, also be used as auxiliary solvents.

As liquid solvents diluents or carriers, there are suitable in the main, aromatic hydrocarbons, such as xylene, toluene or alkyl napthalenes, chlorinated aromatic or chlorinated aliphatic hydrocarbons, such as chlorobenzenes, chloroethylenes or methylene chloride, aliphatic hydrocarbons, such as cyclohexane or paraffins, for example mineral oil fractions, alcohols, such as butanol or glycol as well as their ethers and esters, ketones, such as acetone, methyl ethyl ketone, methyl-isobutyl. ketone or cyclohexanone, or strongly polar solvents, such as dimethylformamide and dimethyl-sulphoxide, as well as water.

By liquefied gaseous diluents or carriers are meant liquids which would be gaseous at normal temperature and under normal pressure, for example aerosol propellants, such as halogenated hydrocarbons as well as butane, propane, nitrogen and carbon dioxide.

As solid carriers there may be used ground natural minerals, such as kaolins, clays, talc, chalk, quartz, attapulgite, montmorillonite or diatomaceous earth, and ground synthetic minerals, such as highly-dispersed silicic acid, alumina and silicates. As solid carriers for granules there may be used crushed and fractionated natural rocks such as calcite, marble, pumice, sepiolite and dolomite, as well as synthetic granules of inorganic and organic meals, and granules of organic material such as sawdust, coconut shells, maize cobs and tobacco stalks.

As emulsifying and/or foam-forming agents there may be used non-ionic and anionic emulsifiers, such as polyoxyethylene-fatty acid esters, polyoxyethylene-fatty alcohol ethers, for example alkylaryl polyglycol ethers, alkyl sulphonates, alkyl sulphates, aryl sulphonates as well as albumin hydrolysis products.

Dispersing agents include, for example, lignin sulphite waste liquors and methylcellulose.

Adhesives such as carboxymethylcellulose and natural and synthetic polymers in the form of powders, granules or latices, such as gum arabic, polyvinyl alcohol and polyvinyl acetate, can be used in the formulation.

It is possible to use colorants such as inorganic pigments, for example iron oxide, titanium oxide and Prussian Blue, and organic dyestuffs, such as alizarin dye stuffs, azo dye stuffs or metal phthalocyanine dye stuffs, and trace nutrients, such as salts of iron, manganese boron, copper, cobalt, molybdenum and zinc.

The active compounds according to the invention, as such or in the form of their formulations, can also be used, for combating weeds, as mixtures with known herbicides, finished formulations or tank mixes being possible.

Mixtures with other known active compounds, such as herbicides, fungicides, insecticides, acaricides, nematicides, bird repellents, plant nutrients and agents which improve soil structure, are also possible.

The active compounds can be used as such, in the form of their formulations or in the use forms prepared therefrom by further dilution, such as ready-to-use solutions, suspensions, emulsions, powders, pastes and granules. They are used in the customary manner, for example by watering, spraying, atomizing or scattering.

The active compounds according to the invention can be applied either before or after emergence of the plants. They can also be incorporated into the soil before sowing. They are used, in particular, after emergence of the plants.

The amount of active compound used can vary within a substantial range. It depends essentially on the nature of the desired effect. In general, the amounts used are between 0.001 and 10 kg of active compound per hectare of soil surface, preferably between 0.01 and 5 kg per ha.

The preparation and use of the active compounds according to the invention can be seen from the following examples, but they should not be regarded as limiting the scope of the invention.

### Synthesis Examples

### Synthesis Example 1

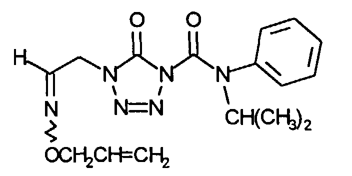

Concentrated hydrochloric acid (20 ml) was added to a tetrahydrofuran solution (30 ml) of 1-(2-dimethoxyethyl)-4-(N-isopropylN-phenylcarbamoyl)-5(4H)tetrazolinone (5.8 g) and the mixture was left to stand for one day. To the oily product (5.1 g) obtained by distilling off the solvent, O-allylhydroxylamine-hydrochloric acid salt (2.4 g), sodium acetate (2.0 g) and ethanol (70 ml) were added and the mixture was heated under reflux for 6 hours. Ethanol was distilled off under reduced pressure. Then, the resulting residue was extracted with ethyl acetate and the ethyl acetate solution was washed with water and then dried. Ethyl acetate was distilled off under reduced pressure and the resulting oily product was subjected to column chromatography on silica gel (n-hexane : ethyl acetate = 2 : 1) to obtain an isomer mixture of 1-(2-allyloxyiminoethyl)-4-(N-isopropyl-N-phenylcarbamoyl)-5(4H)tetrazolinone (3.1 g) as an oily product;
refractive index (n_{D}²⁰) = 1.5305

### Synthesis Example 2

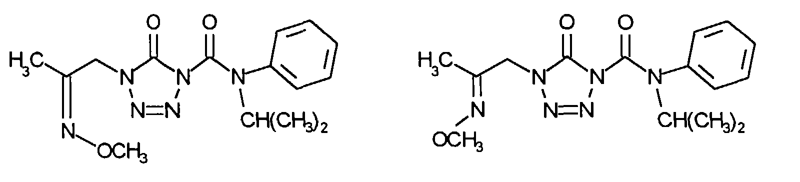

1-(acetonyl)-4-(N-isopropyl-N-phenylcarbamoyl)-5(4H)tetrazolinone (0.8 g), O-methylhydroxylamine-hydrochloric acid salt (0.4 g) and sodium acetate (0.4 g) were dissolved in ethanol (50 ml) and the mixture was heated under reflux for 4 hours. The reaction solution was filtered under suction and the crystals were filtered off. The filtrate was distilled off under reduced pressure and the resulting crude product was extracted with dichloromethane. This was washed with water and dried over anhydrous magnesium sulfate. Thereafter, the solvent was distilled off under reduced pressure and the residue was purified by column chromatography on silica gel (ethyl acetate : chloroform = 1 : 100) to obtain firstly Syn-1-(2-methoxyiminopropyl)-4-(N-isopropyl-N-phenylcarbamoyl)-5(4H)tetrazolinone (0.4 g; melting point: 82-84 °C) and then Anti-1-(2-methoxyiminopropyl)-4-(N-isopropyl-N-phenylcarbamoyl)-5(4H)-tetrazolinone (0.3 g; melting point: 84.5-92°C). The Rf value of the Syn form was 0.3 and the Rf value of the Anti form was 0.2.

### Synthesis Example 3

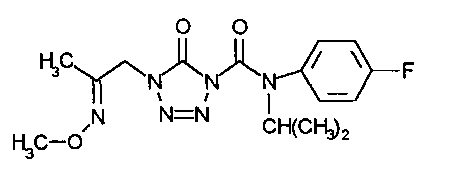

4-Dimethylaminopyridine (0.7 g) was added to a toluene solution of the Anti form of 1-(2-methoxyiminopropyl)-5(4H)-tetrazolinone (0.7 g) and N-(4-fluorophenyl)-N-isopropylcarbamolyl chloride (0.9 g) at room temperature and then the mixture was reacted at about 60°C for 8 hours. The reaction solution was washed successively with water, diluted hydrochloric acid and water and dried over anhydrous magnesium sulfate. The solvent was distilled off and the resulting residue was purified by column chromatography on silica gel (ethyl acetate : chloroform = 1 : 100) to obtain the Anti form of 1-(2-methoxyiminopropyl)-4-N-(4-fluorophenyl)-N-isopropylcarbamoyl-5(4H)-tetrazolinone (1.2 g) as crystals;
melting point: 82.5-83.5°C

Tables 4 and 5 show the compounds obtained in the same manner as those of the above Synthesis Examples 1 to 3. Table 4 also shows the compounds of the above Synthesis Examples 1 to 3.

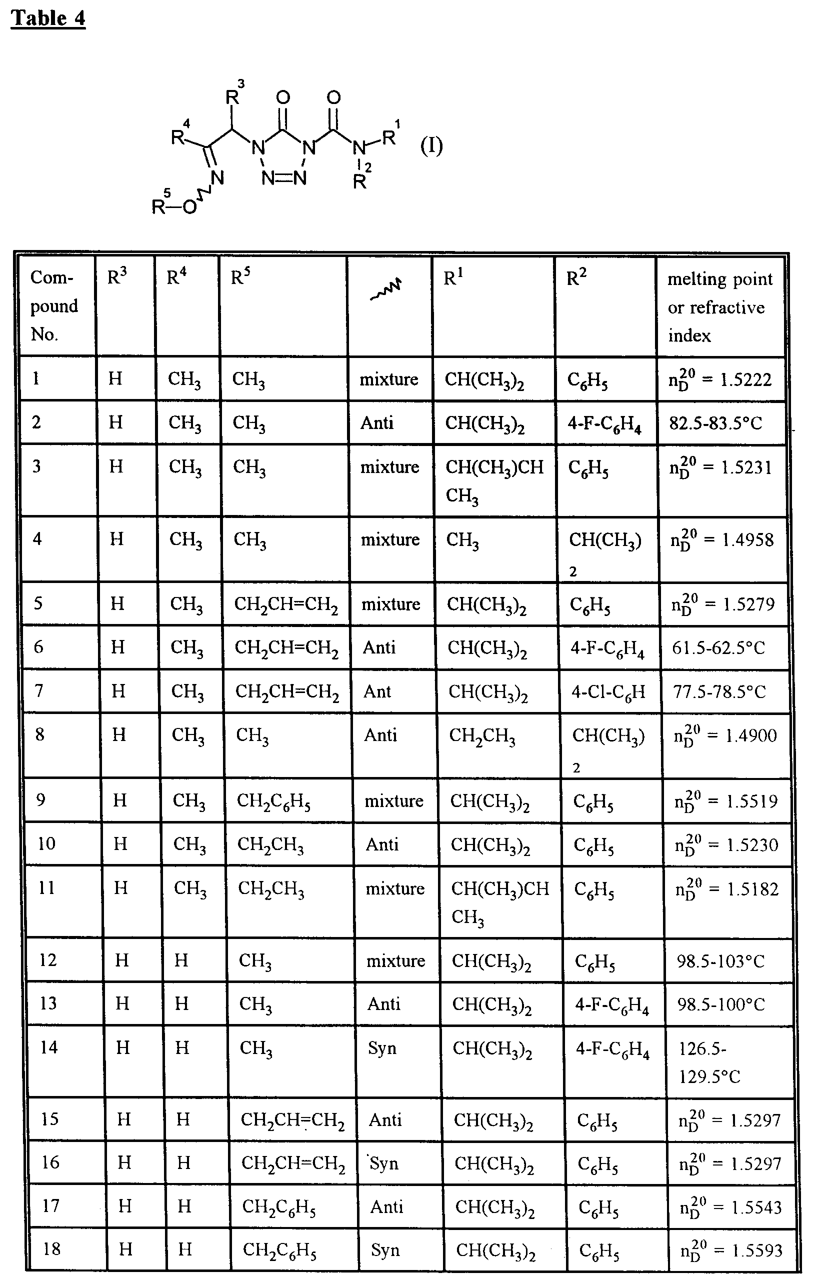

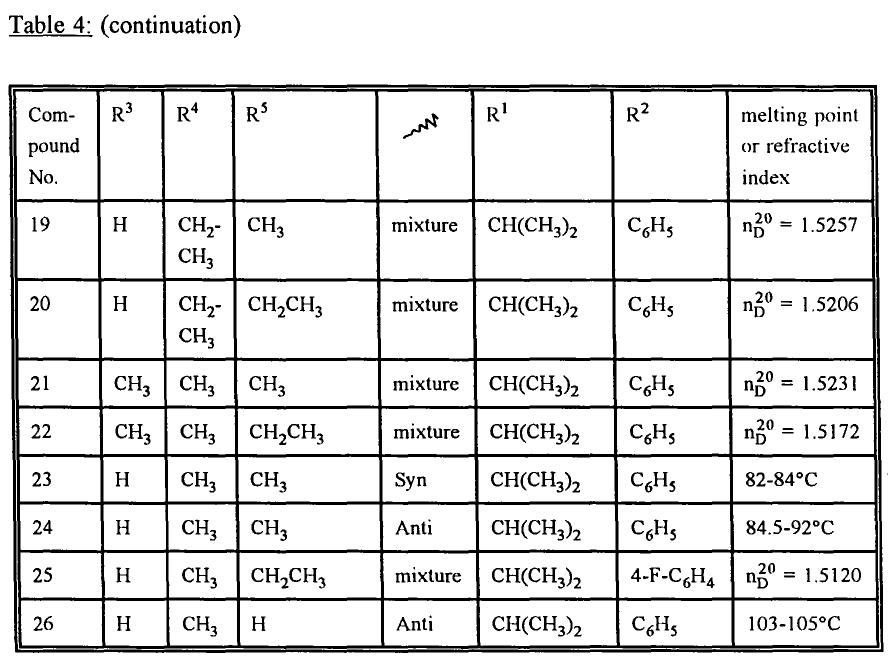

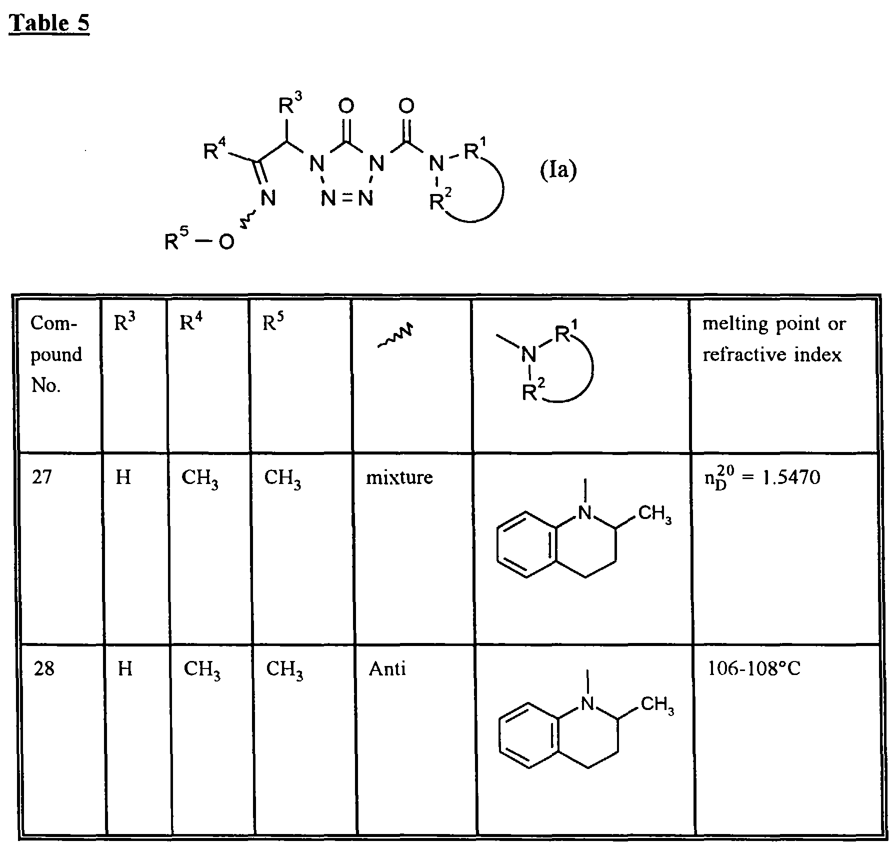

### Synthesis of intermediates:

### Synthesis Example 4

### (Synthesis of the starting material for producing the compound of Synthesis Example 1)

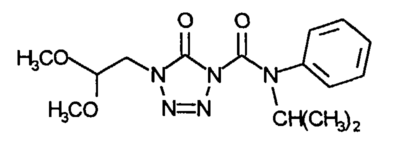

To an aqueous solution (5 ml) of sodium hydroxide (2.2 g), a methanol solution (50 ml) of 1-(2,2-dimthoxyethyl)-(4H)-tetrazolin-5-thione (8.4 g) was added and then propylene oxide (3.4 g) was added while stirring and cooling with ice, and the mixure was left to stand at room temperature overnight. The aqueous solution obtained by distilling off methanl was washed with ethyl acetate and then water was distilled off under reduced pressure. To the residue, anhydrous methanol was added and the solution was acidified by introducing hydrochlic acid gas at room temperature. Thereafter, methanol was distilled off under reduced pressure and the resulting residue was extracted with ethyl acetate. Then, to a toluene solution of the residue (6.3 g) obtained by distilling off ethyl acetate and N-isopropyl-N-phenylcarbamoyl chloride (7.2 g), 4-dimethylaminopyridine was added and the mixture was reacted at about 50°C for 6 hours. The reaction solution was washed with water, diluted hydrochloric acid and water, dried over magnesium sulfate and toluene was distilled off. Thereafter, the resulting residue was purified by column chromatography on silica gel (ethyl acetate : hexane = 1 : 3) to obtain 1-(2,2-dimethoxyethyl)-4-(N-isopropyl-N-phenyl- carbamoyl)-5(4H)-tetrazolinone (7.1 g) as an oily product.
refractive index (n_{D}²⁰) = 1.5160

### Synthesis Example 5

### (Synthesis of the starting material for producing the compound of Synthesis Example 2)

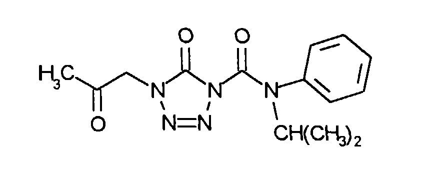

4-Dimethylaminopyridine (6.8 g) was added to a toluene solution of 1-acetonyl-5(4H)-tetrazolinone (5 g) and N-isopropyl-N-phenylcarbamoyl chloride (7.3 g) and the mixture was reacted at 65°C for 10 hours. The reaction solution was filtered under suction, the filtrate was washed with water, diluted hydrochloric acid and water and then dried over anhydrous magnesium sulfate. Thereafter, the solvent was distilled off under reduced pressure and the resulting residue was purified by column chromatography on silica gel (ethyl acetate : chloroform = 1 : 25) to obtain 1-(acetonyl)-4-(N-isopropyl-N-phenylcarbamoyl)-5(4H)-tetrazolinone (9.1 g);
refractive index (n_{D}²⁰) = 1.5228

Further, 1-(2-oxopropyl)-4-(2-methyl-1,2,3,4-tetrahydroquinolin-1-ylcarbonyl)-5(4H)-tetrazolinone was obtained in a manner similar to that of Synthesis Example 5.
melting point: 103-105°C

### Synthesis Example 6

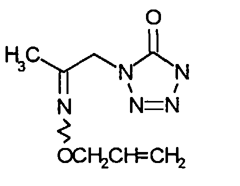

An ethanol solution of 1-acetonyl-5(4H)-tetrazolinone (1.4 g), O-allylhydroxylamine hydrochloric acid salt (2.4 g) and sodium acetate (1.8 g) was heated under reflux for 4 hours. Ethanol was distilled off under reduced pressure and the residue was extracted with ethyl acetate. The ethyl acetate-extraction product was subjected to column chromatography on silica gel (ethyl acetate : hexane = 1 : 1) to obtain 1-(2-allyloxyiminopropyl)-tetrazolin-5(4H)-one (Anti : Syn = 5 : 1) (1.8 g);
melting point: 57-60.5°C

Further, 1-(2-methoxyiminopropyl)-tetrazolin-5(4H)-one (Anti form) (melting point: 99.5-101°C) was obtained as the main product in a manner similar to that of Synthesis Example 6.

### Synthesis Example 7

### (Synthesis of the starting material for producing the compound of Synthesis Example 6)

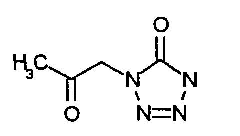

A catalytic amount of boron trifluoride-ether complex was added to a mixed solution of 2,2,6-trimethyl-4H-1,3-dioxin-4-one (14.2 g) and trimethylsilyl azide (25.0 g) at room temperature and the mixture was reacted at 100 - 110°C for 6 hours. The unreacted trimethylsilyl azide was distilled off under reduced pressure, the resulting residue was dissolved in methanol by the addition of methanol, and then methanol was distilled off under reduced pressure. The residue was subjected to column chromatography on silica gel (ethyl acetate) to obtain 1-acetonyl-5(4H)-tetrazolinone (10.6 g) as colorless crystals.
melting point: 120-122°C

Further, 1-(2-oxobutyl)-5(4H)-tetrazolinone (melting point: 99-111.5°C) was obtained as colorless crystals in a manner similar to that of Synthesis Example 7.

### Synthesis Example 8

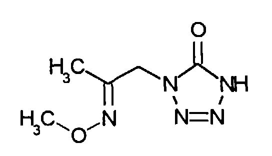

To an aqueous solution (water: 5 ml) of sodium hydroxide (2.3 g), a methanol solution of 1-(2,2-dimethoxyethyl)-4H-tetrazolin-5-thione (8.7 g) was added and then propylene oxide (3.5 g) was added while stirring and cooling with ice. Then, the mixture was left to stand at room temperature overnight. After distilling off methanol under reduced pressure, this was acidified with concentrated hydrochloric acid, followed by the extraction with a methanol : chloroform (1 : 10) solution. To the residue (6.1 g) obtained by distilling off the solvent, O-methylhydroxylamine- hydrochloric acid salt (8.6 g), sodium acetate (8.6 g) and ethanol were added and the mixture was heated under reflux for 4 hours. Ethanol was distilled off under reduced pressure and the resulting oily product was subjected to column chromatography on silica gel (ethyl acetate : hexane = 1 : 1) to obtain the Anti form of 1-(2-methoxyiminoethyl)-5(4H)-tetrazolinone (3.3 g) as the main product;
melting point: 115-116.5°C

### Synthesis Example 9

### (Synthesis of the starting material for producing the compound of Synthesis Example 8)

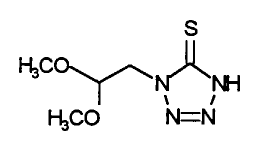

An aqueous solution of methyl N-(2,2-dimethoxyethyl)dithiocarbamate (9.8 g) and sodium azide (3.7 g) was heated to 100°C. After the completion of evolution of methylmercaptan, the reaction solution was cooled to room temperature ad washed with ethyl acetate. Ethyl acetate was poured onto the aqueous solution. Then, the acidic material obtained by acidifying with diluted hydrochloric acid was extracted with ethyl acetate. The ethyl acetate solution was washed with saturated saline, dried over magnesium sulfate, and then ethyl acetate was distilled off to obtain 1-(2,2-dimethoxyethyl)-(4H)-tetrazolin-5- thione (8.4 g).
refractive index (n_{D}²⁰) = 1.5264

### Synthesis Example 10

### (Synthesis of the starting material for producing the compound of Synthesis Example 9)

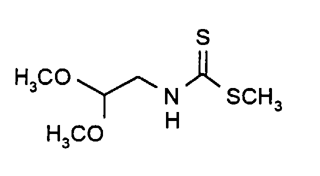

Sodium tert-butoxide (17.1 g) was added to a methanol solution of 2,2-dimethoxyethylamine (14.5 g) and carbon disulfide (15.0 g) while stirring and cooling with ice and the mixture was reacted for 30 minutes. Then dimethyl sulfate (17.5g) was added and the mixture was reacted for 1 hour under cooling with ice. After the completion of reaction, water was added to the reaction solution and then toluene was distilled off under reduced pressure to obtain methyl N-(2,2-dimethoxyethyl)dithiocarbamate (22.5 g) as a yellow oily product. This was used for the reaction of Synthesis Example 9 without purification.

### Test Examples

### Test Example 1

### (Test of pre-emergence soil treatment against plowed land weeds)

### Preparation of testing formulations of active ingredients

- carrier:: acetone 5 parts by weight
- emulsifier:: benzyloxy polyglycol ether 1 part by weight

The formulations of active ingredients are obtained by mixing 1 part by weight of the active compounds and the above amounts of carrier and emulsifier. The prescribed amount of the formulation is diluted with water to prepare a testing formulation.

### Testing method

In the greenhouse, seeds of Echinochloa crus-galli and Amaranthus lividus were sowed each in the surface layer of plowed land soil filled in a 120 cm² pot with soil-covering and each a prescribed amount of the testing formulation prepared by the above method was uniformly spread on the surface layer of soil in the testing pot.
The extent of herbicidal activity was examined after 4 weeks from sowing.

In this test, for example, the active compounds of Compound Nos. 1, 2, 14, 21, 23 and 25 of the invention (see Table 4 above) exhibited a herbicidal activity of 100% against Echinochloa crus-galli and Amaranthus lividus by application of 1.0kg/ha of each active compound.

### Test Example 2

### (Test of post-emergence foliar treatment against plowed land weeds)

### Testing method

In the greenhouse, seeds of Echinochloa crus-galli and Amaranthus lividus were sowed each in a 120 cm² pot filled with plowed land soil and covered with soil. After 10 days from sowing and soil-covering (when the weeds were in 2-foliage stage on average), each a prescribed amount of the formulation prepared similarly to those in the above Test Example 1 was uniformly spread on the foliage part of tested plant in the testing pot. After 3 weeks from spreading, the extent of herbicidal activity was examined.

In this test, for example, the active compounds of Compound Nos. 1, 10, 13, 21 and 25 of the invention (see Table 4 above) exhibited a herbicidal activity of 90% or higher against Echinochloa crus-galli and Amaranthus lividus by application of 2.0 kg/ha of each active compound.

### Test Example 3

### (Herbicidal effect test against paddy field weeds)

### Preparation of formulations of active ingredients

- carrier:: acetone 5 parts by weight
- emulsifier:: benzyloxy polyglycol ether 1 part by weight

The formulations of active ingredients are obtained by mixing 1 part by weight of the active compounds and the above amounts of carrier and emulsifier. The prescribed amount of the formulation is diluted with water to prepare a testing formulation.

### Testing procedure

In the greenhouse, each 3 seedlings of paddy rice (cultivar: Nipponbare) of 2.5 leaf stage (15 cm tall) were transplanted in two places in 1/2000 are large pot (25 x 25 x 9 cm) filled with paddy field soil and saturated with water. Then, seeds of barnyardgrass, small-flower, Monochoria, broad-leaved weeds (*Lindernia pyxidaria, Rotala indica, Elatine triandra, ammannia multiflora Roxb. Dopatrium junceum* Hamilt) and bulrush and a tuber of Japanese ribbon wapato were sowed, and water was poured on the soil to a depth of about 2-3 cm. Each a prescribed amount of the formulation of active compound prepared similarly to those in the above preparation method, was applied to the surface of the water 7 days after the transplanting of the paddy rice.

The herbicidal activity and the degree of phytotoxicity against crop plants were examined on the day after 3 weeks from the treatment during which period the water depth of 3 cm was maintained. The herbicidal activity was rated as 100% in the case of complete death and as 0% in the case where no herbicidal effect was observed or in the case where no phytotoxicity was observed.

In this test, for example, the active compounds of Compound Nos. 1, 2, 6, 9, 13, 14, 22, 23 and 24 of the invention (see Table 4 above) exhibited a herbicidal activity of 90% or higher against barnyardgrass, small-flower, Monochoria, broad-leaved weeds, bulrush and Japanese ribbon wapato by application of 0.5 kg/ha of each active compound.

### Formulation Examples

### Formulation Example 1 (granules)

Water (25 parts) is added to a mixture of Compound No. 3 (10 parts) (for "Compound No." see Table 4 above), bentonite (montmorillonite) (30 parts), talc (58 parts) and lignin sulphonate salt (2 parts) with well kneading and formed in 10-40 mesh granules using an extrusion-type granulator followed by drying at 40-50°C to give granules.

### Formulation Example 2 (granules)

A clay mineral (95 parts) having a particle size distribution within 0.2-2 mm range is introduced in a rotary mixer and Compound No. 1 (5 parts) (for "Compound No." see Table 4 above) is sprayed therein with a liquid diluent under rotation to uniformly wet followed by drying at 40-50°C giving granules.

### Formulation Example 3 (emulsion)

An emulsion is obtained by mixing Compound No. 3 (30 parts) (for "Compound No." see Table 4 above), xylene (5 parts), polyoxyethylene alkyl phenyl ether (8 parts) and calcium alkylbenzene sulphonate (7 parts) with stirring.

### Formulation Example 4 (wettable powder)

A wettable powder is prepared by mixing Compound No. 5 (15 parts) (for "Compound No." see Table 4 above), a mixture (1 : 1) (80 parts) of White Carbon (fine powder of hydrated non-crystalline silicon oxide) and powdery clay, sodium alkylbenzene sulphonate (2 parts) and a condensate of sodium alkylnaphthalene sulphonate and formaldehyde (3 parts) in a powdery state.

### Formulation Example 5 (wettable granules)

Wettable granules are prepared by thoroughly mixing Compound No. 2 (20 parts) (for "Compound No." see Table 4 above), sodium lignin sulphonate (30 parts), bentonite (15 parts) and calcined diatomaceous earth powder (35 parts) followed by addition of water and extrusion through a 0.3 mm screen and drying.

## Claims

1. Compounds of the formula: 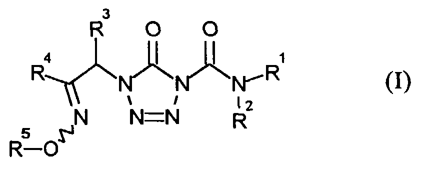 wherein
R¹ and R², independently of one another, are C₁₋₆ alkyl, C₁₋₆ haloalkyl, C₃₋₈ cycloalkyl, C₂₋₆ alkenyl, C₂₋₆ haloalkenyl, C₃₋₆ alkynyl or optionally substituted phenyl, or
R¹ and R², together with the nitrogen atom to which they are bonded, form a 5-membered or 6-membered heterocyclic ring which may be benzo-fused or which may be substituted by halogen or C₁₋₄ alkyl,
R³ is hydrogen or C₁₋₆ alkyl,
R⁴ is hydrogen or C₁₋₆ alkyl, or
R³ and R⁴, together with the carbon atoms to which they are bonded, form cyclopentylidene or cyclohexylidene,
R⁵ is C₁₋₆ alkyl, C₃₋₆ alkenyl or benzyl, and
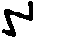 is a single bond of Anti form or of Syn form.

2. The compounds of formula (I) according to claim 1,
wherein
R¹ and R², independently of one another, are C₁₋₄ alkyl, C₁₋₄ haloalkyl, cyclopropyl, cyclopentyl, cyclohexyl, C₂₋₄ alkenyl, C₂₋₄ haloalkenyl or C₃₋₄ alkynyl, or represent phenyl which may be substituted by halogen or C₁₋₄ alkyl, or
R¹ and R², together with the nitrogen atom to which they are bonded, form pyrrolidin-1-yl, 2,5-dimethylpyrrolidin-1-yl, 3-pyrrolin-1-yl, 2,5-dimethyl-3-pyrrolin-1-yl, piperidino, 2-methylpiperidino, 2,6-dimethylpiperidino, piperazin-1-yl, morpholino, 1,2,3,4-tetrahydroquinolin-1-yl, 2-methyl-1,2,3,4-tetrahydroquinolin-1-yl, 2,2-dimethyl-1,2,3,4-tetrahydroquinolin-1-yl, 2,2-dimethyl-1,2-dihydroquinolin-1-yl or 6-fluoro-2,2-dimethyl-1,2-dihydroquinolin-1-yl,
R³ is hydrogen or C₁₋₄ alkyl,
R⁴ is hydrogen or C₁₋₄ alkyl, or
R³ and R⁴, together with the carbon atoms to which they are bonded, form cyclopentylidene or cyclohexylidene,
R⁵ is C₁₋₄ alkyl, C₂₋₄ alkenyl or benzyl, and
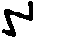 is a single bond of Anti form or of Syn form.

3. The compounds of formula (I) according to claims 1 or 2,
wherein,
R¹ and R² independently of one another represent C₁₋₄ alkyl, C₁₋₄ haloalkyl, cyclopropyl, cyclopentyl, cyclohexyl, C₂₋₄ alkenyl, C₂₋₄ haloalkenyl or C₃₋₄ alkynyl, or represent phenyl which may be substituted by methyl, fluoro, chloro or bromo, or
R¹ and R², together with the nitrogen atom to which they are bonded, form pyrrolidin-1-yl, 2,5-dimethylpyrrolidin-1-yl, 3-pyrrolin-1-yl, 2,5-dimethyl-3-pyrrolin-1-yl, piperidino, 2-methylpiperidino, 2,6-dimethylpiperidino, piperazin-1-yl, morpholino, 1,2,3,4-tetrahydroquinolin-1-yl, 2-methyl-1,2,3,4-tetrahydroquinolin-1-yl, 2,2-dimethyl-1,2,3,4-tetrahydroquinolin-1-yl, 2,2-dimethyl-1,2-dihydroquinolin-1-yl or 6-fluoro-2,2-dimethyl-1,2-dihydroquinolin-1-yl,
R³ represents hydrogen or methyl,
R⁴ represents hydrogen, methyl or ethyl, or
R³ and R⁴, together with the carbon atoms to which they are bonded, form cyclopentylidene or cyclohexylidene,
R⁵ represents methyl, ethyl, allyl or benzyl, and
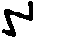 represents a single bond of Anti form or of Syn form.

4. Processes for the preparation of the compounds and mixtures of geometrical isomers, of the formula (I): 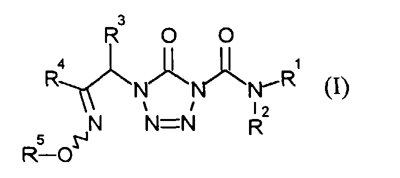 wherein
R¹ and R², independently of one another, are C₁₋₆ alkyl, C₁₋₆ haloalkyl, C₃₋₈ cycloalkyl, C₂₋₆ alkenyl, C₂₋₆ haloalkenyl, C₃₋₆ alkynyl or optionally substituted phenyl, or
R¹ and R², together with the nitrogen atom to which they are bonded, form a 5-membered or 6-membered heterocyclic ring which may be benzo-fused or which may be substituted by halogen or C₁₋₄ alkyl,
R³ is hydrogen or C₁₋₆ alkyl,
R⁴ is hydrogen or C₁₋₆ alkyl, or
R³ and R⁴, together with the carbon atoms to which they are bonded, form cyclopentylidene or cyclohexylidene,
R⁵ is C₁₋₆ alkyl, C₃₋₆ alkenyl or benzyl, and
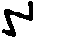 is a single bond of Anti form or of Syn form,
characterized in that
(a):
compounds of the formula (II): 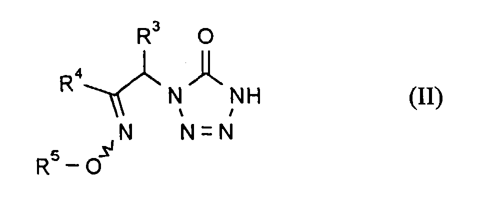 wherein
R³, R⁴ and R⁵ have the above-mentioned meanings,
are reacted with compounds of the formula (III): 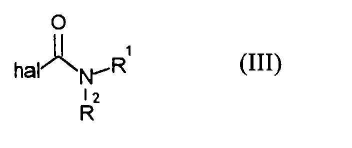 wherein
R¹ and R² have the above-mentioned meanings, and hal is a leaving group such as a chlorine atom or bromine atom,
in the presence of inert solvents, and if appropriate, in the presence of an acid binder,
or
(b)
compounds of the formula (IV): 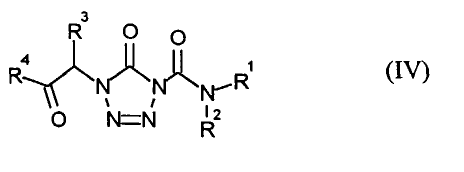 wherein
R¹, R², R³ and R⁴ have the above-mentioned meanings,
are reacted with compounds of the formula (V):
R⁵―O―NH₂ (V)
wherein
R⁵ has the above-mentioned meaning,
in the presence of inert solvents, and if appropriate, in the presence of an acid binder.

5. Herbicidal compositions, characterized in that they contain at least one compound of formula (I) according to claim 1.

6. Process for combating weeds, characterized in that compounds of formula (I) according to claim 1 are allowed to act on weeds and/or their habitat.

7. Use of compounds of formula (I) according to claim 1 for combating weeds.

8. Process for the preparation of herbicidal compositions, characterized in that compounds of formula (I) according to claim 1 are mixed with extenders and/or surface active agents.

9. Compounds of the formula (X): 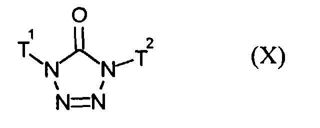 wherein
T¹ is hydrogen, 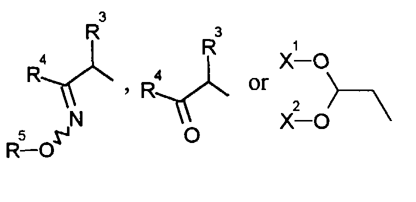
T² is hydrogen or 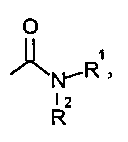 with the proviso that when T¹ is 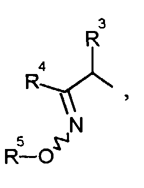 then
T² is hydrogen,
R¹ and R² independently of one another are C₁₋₆ alkyl, C₁₋₆ haloalkyl, C₃₋₈ cycloalkyl, C₂₋₆ alkenyl, C₂₋₆ haloalkenyl, C₃₋₆ alkynyl or optionally substituted phenyl, or
R¹ and R², together with the nitrogen atom to which they are bonded, form a 5-membered or 6-membered heterocyclic ring which may be benzo-fused or which may be substituted by halogen or C₁₋₄ alkyl,
R³ is hydrogen or C₁₋₆ alkyl,
R⁴ is hydrogen or C₁₋₆ alkyl, or
R³ and R⁴, together with the carbon atoms to which they are bonded, form cyclopentylidene or cyclohexylidene,
R⁵ is C₁₋₆ alkyl, C₃₋₆ alkenyl or benzyl,
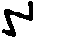 is a single bond of Anti form or of Syn form, and
X¹ and X² independently of one another are hydrogen or C₁₋₄ alkyl.

10. The compounds of formula (X) according to claim 9,
wherein
T¹ is hydrogen, 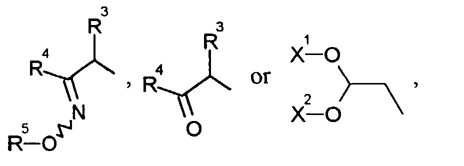
T² is hydrogen or 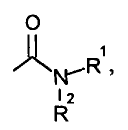 with the proviso that when T¹ is 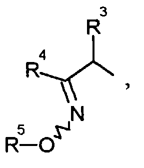 then
T² is hydrogen,
R¹ and R² independently of one another are C₁₋₄ alkyl, C₁₋₄ haloalkyl, cyclopropyl, cyclopentyl, cyclohexyl, C₂₋₄ alkenyl, C₂₋₄ haloalkenyl or C₃₋₄ alkynyl, or are phenyl which may be substituted by halogen or C₁₋₄ alkyl, or
R¹ and R², together with the nitrogen atom to which they are bonded, form pyrrolidin-1-yl, 2,5-dimethylpyrrolidin-1-yl, 3-pyrrolin-1-yl, 2,5-dimethyl-3-pyrrolin-1-yl, piperidino, 2-methylpiperidino, 2,6-dimethylpiperidino, piperazin-1-yl, morpholino, 1,2,3,4-tetrahydroquinolin-1-yl, 2-methyl-1,2,3,4-tetrahydroquinolin-1-yl, 2,2-dimethyl-1,2,3,4-tetrahydroquinolin-1-yl, 2,2-dimethyl-1,2-dihydroquinolin-1-yl or 6-fluoro-2,2-dimethyl-1,2-dihydroquinolin-1-yl,
R³ is hydrogen or C₁₋₄ alkyl,
R⁴ is hydrogen or C₁₋₄ alkyl, or
R³ and R⁴, together with the carbon atoms to which they are bonded, form cyclopentylidene or cyclohexylidene,
R⁵ is C₁₋₄ alkyl, C₂₋₄ alkenyl or benzyl,
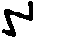 is a single bond of Anti form or of Syn form, and
X¹ and X² independently of one another are hydrogen or C₁₋₃ alkyl.

11. The compounds of formula (X) according to claim 9,
wherein
T¹ is hydrogen, 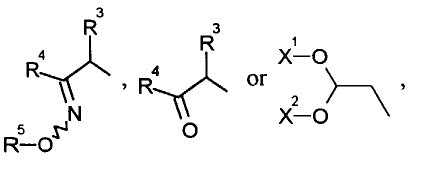
T² is hydrogen or 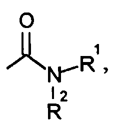 with the proviso that when
T¹ represents 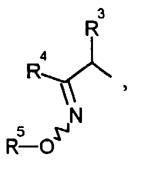 then
T² represents hydrogen,
R¹ and R² independently of one another are C₁₋₄ alkyl, C₁₋₄ haloalkyl, cyclopropyl, cyclopentyl, cyclohexyl, C₂₋₄ alkenyl, C₂₋₄ haloalkenyl or C₃₋₄ alkynyl, or represent phenyl which may be substituted by methyl, fluoro, chloro or bromo, or
R¹ and R², together with the nitrogen atom to which they are bonded, form pyrrolidin-1-yl, 2,5-dimethylpyrrolidin-1-yl, 3-pyrrolin-1-yl, 2,5-dimethyl-3-pyrrolin-1-yl, piperidino, 2-methylpiperidino, 2,6-dimethylpiperidino, piperazin-1-yl, morpholino, 1,2,3,4-tetrahydroquinolin-1-yl, 2-methyl-1,2,3,4-tetrahydroquinolin-1-yl, 2,2-dimethyl-1,2,3,4-tetrahydroquinolin-1-yl, 2,2-dimethyl-1,2-dihydroquinolin-1-yl or 6-fluoro-2,2-dimethyl-1,2-dihydroquinolin-1-yl,
R³ is hydrogen or methyl,
R⁴ is hydrogen, methyl or ethyl, or
R³ and R⁴, together with the carbon atoms to which they are bonded, form cyclopentylidene or cyclohexylidene,
R⁵ is methyl, ethyl, allyl or benzyl,
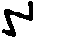 is a single bond of Anti form or of Syn form, and
X¹ and X² independently of one another are hydrogen or methyl.
